# EUROPEAN PATENT APPLICATION

(11) **EP 4 582 411 A1**
(43) Date of publication of application: **09.07.2025**
(21) Application number: 23860152.0
(22) Date of filing: 23.08.2023
(51) Int. Cl.: C07C 319/20, C07C 211/27, C07C 321/14, C08G 18/38, C08J 11/24, C08J 11/28, G02B 1/00, G02B 1/04

(54) **METHOD FOR PRODUCING POLYTHIOL COMPOSITION, METHOD FOR PRODUCING POLYAMINE COMPOUND AND APPLICATIONS OF THESE**

(30) Priority: 29.08.2022 JP 2022135892
(71) Applicant: Mitsui Chemicals, Inc., Tokyo 104-0028 (JP)
(72) Inventor: NAKAI, Shinnosuke, Omuta-shi, Fukuoka 836-8610 (JP); MURAKAMI, Masakazu, Omuta-shi, Fukuoka 836-8610 (JP)
(74) Representative: Mewburn Ellis LLP
(86) International application number: PCT/JP2023/030285
(87) International publication number: WO 2024/048389

(57) **Abstract**

A method of producing a polythiol composition, comprising a generation step of generating a polythiol composition by decomposing a thiourethane resin using a decomposing agent represented by the following Formula (1) or (2). In Formula (1), R¹ and R² each independently represent a hydrogen atom, an alkyl group having from 1 to 3 carbon atoms, or an amino group, excluding a case in which, and in Formula (2), R³ represents an alkyl group having from 1 to 3 carbon atoms.

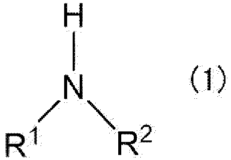

R³-OH (2)

## Description

### Technical Field

The present disclosure relates to a method of producing a polythiol composition, a method of producing a polyamine compound, and applications thereof.

### Background Art

Plastic lenses, which are lenses containing resin, are lighter and less likely to break than inorganic lenses, and can be dyed, and therefore have rapidly become more widely used in recent years for applications such as eyeglass lenses and camera lenses.

For example, various studies have been conducted on lenses containing a thiourethane resin (see, for example, Patent Literature 1 to 3).

There is also a known method of producing a thiourethane resin raw material, in which a thiourethane resin raw material (i.e., a raw material for producing a thiourethane resin, such as a polythiol composition or a polyamine compound) is produced using a thiourethane resin as a starting raw material.

For example, Patent Literature 4 discloses a method of producing a polythiol composition, including a reaction step of reacting a thiourethane resin with an amine compound to produce a polythiol composition.

In addition, Patent Literature 5 discloses a method of producing a polyamine compound, including: a first step of reacting a thiourethane resin with an amine compound A to produce a polyurea compound; and a second step of reacting the polyurea compound with an amine compound B to produce a polyamine compound.

Patent Literature 5 also discloses a method of producing a polyamine compound, including: Step X1 of reacting a thiourethane resin with an alcohol compound in the presence of an amine compound XA, which is a tertiary amine compound, to produce a polycarbamate compound; and Step X2 of reacting the polycarbamate compound with an amine compound XB to produce a polyamine compound.
Patent Literature 1: Japanese Patent Application Laid-Open (JP-A) No. S63-46213
Patent Literature 2: JP-ANo. H2-270859
Patent Literature 3: JP-A No. H7-252207
Patent Literature 4: WO 2021/157701
Patent Literature 5: WO 2021/157702

### SUMMARY OF INVENTION

### Technical Problem

In the production methods of Patent Literature 4 and 5, an amine compound or an alcohol compound is used as a decomposing agent for decomposing a thiourethane resin, and the thiourethane resin is decomposed by the decomposing agent to obtain a polythiol composition or a polyamine compound as a target product.

In the production methods of Patent Literature 4 and 5, it may be required to further improve the removability when removing a decomposing agent (i.e., an amine compound or an alcohol compound) from a reaction system after a decomposition reaction in which a thiourethane resin is decomposed by the decomposing agent.

An object of one aspect of the disclosure is to provide a method of producing a polythiol composition, which exhibits an excellent ability to remove a decomposing agent from a decomposition reaction system after a reaction of decomposing a thiourethane resin by the decomposing agent, a method of producing a polyamine compound, and applications thereof.

### Solution to Problem

Means for solving the above problems include the following aspects.
<1> A method of producing a polythiol composition, the method comprising a generation step of generating a polythiol composition by decomposing a thiourethane resin using a decomposing agent represented by the following Formula (1) or (2):

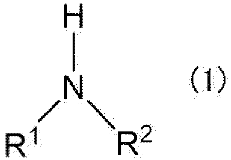

   R³-OH (2)

   wherein, in Formula (1), each of R¹ and R² independently represents a hydrogen atom, an alkyl group having from 1 to 3 carbon atoms, or an amino group, excluding a case in which R¹ and R² are both an amino group, and
   in Formula (2), R³ represents an alkyl group having from 1 to 3 carbon atoms.
<2> The method of producing a polythiol composition according to <1>, wherein the generation step comprises reacting the thiourethane resin with the decomposing agent under a pressure higher than atmospheric pressure.
<3> The method of producing a polythiol composition according to <1> or <2>, which is a method of producing a polythiol composition for use in producing an optical material.
<4> The method of producing a polythiol composition according to any one of <1> to <3>, wherein the thiourethane resin is recovered during at least one of a process of manufacturing eyeglass lenses, a process of manufacturing eyeglasses, or a process of disposing of eyeglasses.
<5> A method of producing a polymerizable composition, the method comprising:
   a step of producing a polythiol composition by the method of producing a polythiol composition according to any one of <1> to <4>; and
   a step of mixing the polythiol composition with a polyisocyanate compound, thereby obtaining a polymerizable composition containing the polythiol composition and the polyisocyanate compound.
<6> The method of producing a polymerizable composition according to <5>, wherein the step of obtaining a polymerizable composition is a step of mixing the polythiol composition with a polyisocyanate composition containing the polyisocyanate compound, thereby obtaining a polymerizable composition containing the polythiol composition and the polyisocyanate composition,
   wherein the polyisocyanate composition contains:
   a xylylene diisocyanate, and
   at least one selected from the group consisting of the following compound (N1), the following compound (N2), and the following compound (N3), and
   wherein, in a case in which the polyisocyanate composition contains the compound (N1), the compound (N1) has a peak area corresponding to 0.20 ppm or more with respect to a peak area of 100 of the xylylene diisocyanate in a measurement by high-performance liquid chromatography,
   in a case in which the polyisocyanate composition contains the compound (N2), the compound (N2) has a peak area corresponding to 0.05 ppm or more with respect to a peak area of 100 of the xylylene diisocyanate in a measurement by high-performance liquid chromatography, or
   in a case in which the polyisocyanate composition contains the compound (N3), the compound (N3) has a peak area corresponding to 0.10 ppm or more with respect to a peak area of 100 of the xylylene diisocyanate in a measurement by high-performance liquid chromatography: 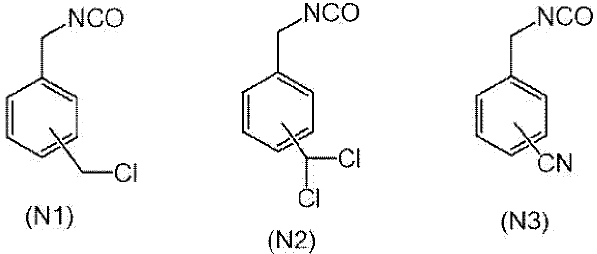
<7> A method of producing a resin, the method comprising:
   a step of producing a polymerizable composition by the method of producing a polymerizable composition according to <5>; and
   a step of obtaining a resin by curing the polymerizable composition.
<8> A method of producing a polyamine compound, the method comprising:
   a first step of generating a polyurea compound by decomposing a thiourethane resin using a decomposing agent represented by the following Formula (1); and
a second step of generating a polyamine compound by decomposing the polyurea compound using a decomposing agent represented by the following Formula (3): 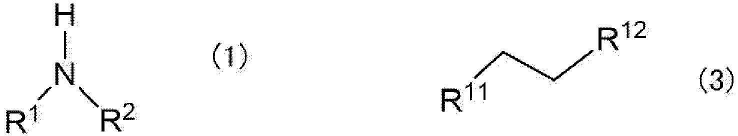
   wherein, in Formula (1), each of R¹ and R² independently represents a hydrogen atom, an alkyl group having from 1 to 3 carbon atoms, or an amino group, excluding a case in which R¹ and R² are both an amino group, and
   in Formula (3), each of R¹¹ and R¹² independently represents a hydroxy group, a mercapto group, an amino group, or a monomethylamino group.
<9> The method of producing a polyamine compound according to <8>, wherein the decomposing agent represented by Formula (3) is ethylenediamine, N,N'-dimethylethylenediamine, 2-aminoethanol, or ethylene glycol.
<10> The method of producing a polyamine compound according to <8> or <9>, wherein the first step comprises reacting the thiourethane resin with the decomposing agent represented by Formula (1) under a pressure higher than atmospheric pressure.
<11> The method of producing a polyamine compound according to any one of <8> to <10>, wherein the second step comprises reacting the polyurea compound with the decomposing agent represented by Formula (3) under a pressure higher than atmospheric pressure.
<12> A method of producing a polyamine compound, the method comprising:
   a step X1 of generating a polycarbamate compound by decomposing a thiourethane resin using a decomposing agent represented by the following Formula (2) in the presence of a tertiary amine compound serving as a decomposition aid; and
   a step X2 of generating a polyamine compound by decomposing the polycarbamate compound using a decomposing agent represented by the following Formula (3): 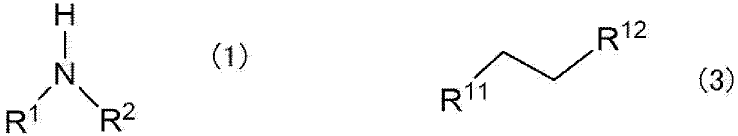
   wherein, in Formula (2), R³ represents an alkyl group having from 1 to 3 carbon atoms, and
   in Formula (3), each of R¹¹ and R¹² independently represents a hydroxy group, a mercapto group, an amino group, or a monomethylamino group.
<13> The method of producing a polyamine compound according to <12>,
   wherein:
   the tertiary amine compound has a molecular weight of 1000 or less, and
   the decomposing agent represented by Formula (3) is ethylenediamine, N,N'-dimethylethylenediamine, 2-aminoethanol, or ethylene glycol.
<14> The method of producing a polyamine compound according to <12> or <13>, wherein the step X1 comprises reacting the thiourethane resin with the decomposing agent represented by Formula (2) under a pressure higher than atmospheric pressure.
<15> The method of producing a polyamine compound according to any one of <12> to <14>, wherein the step X2 comprises reacting the polycarbamate compound with the decomposing agent represented by Formula (3) under a pressure higher than atmospheric pressure.
<16> The method of producing a polyamine compound according to any one of <8> to <15>, which is a method of producing a polyamine compound as a raw material for a polyisocyanate compound for use in producing an optical material.
<17> The method of producing a polyamine compound according to any one of <8> to <15>, wherein the thiourethane resin is recovered during at least one of a process of manufacturing eyeglass lenses, a process of manufacturing eyeglasses, or a process of disposing of eyeglasses.
<18> A method of producing a polyisocyanate compound, the method comprising:
   a step of producing a polyamine compound by the method of producing a polyamine compound according to any one of <8> to <16>; and
   a step of reacting at least one of the polyamine compound or a hydrochloride of the polyamine compound with carbonyl dichloride, thereby obtaining a polyisocyanate compound.
<19> A method of producing polymerizable composition, the method comprising:
   a step of producing a polyisocyanate compound by the method of producing a polyisocyanate compound according to <18>; and
   a step of mixing at least the polyisocyanate compound with an active hydrogen compound, thereby obtaining a polymerizable composition containing the polyisocyanate compound and the active hydrogen compound.
<20> A method of producing a resin, the method comprising:
   a step of producing a polymerizable composition by the method of producing a polymerizable composition according to <19>; and
   a step of obtaining a resin by curing the polymerizable composition.

### Advantageous Effects of Invention

According to the disclosure, a method of producing a polythiol composition, which exhibits an excellent ability to remove a decomposing agent from a decomposition reaction system after a reaction of decomposing a thiourethane resin using the decomposing agent, a method of producing a polyamine compound, and applications thereof are provided.

### DESCRIPTION OF EMBODIMENTS

In the disclosure, a numerical range expressed using "to" means a range that includes the numerical values before and after "to" as the lower and upper limits.

In the disclosure, the term "step" refers not only to an independent step, but also to a step that cannot be clearly distinguished from other steps, as long as the intended purpose of the step is achieved.

In the disclosure, when a plurality of substances corresponding to each component are present in the composition, the amount of each component contained in the composition means the total amount of the plurality of substances present in the composition, unless otherwise specified.

In the disclosure, the upper or lower limit value described in one numerical range may be replaced with the upper or lower limit value of another numerical range described in stages, within numerical ranges described in stages. In addition, the upper or lower limit value within a numerical range described in the disclosure may be replaced with a value indicated in the Examples.

There may be overlaps among a plurality of embodiments in the disclosure. In other words, features of one embodiment may be included in other embodiments.

### <<First Embodiment>>

### [Method of Producing Polythiol Composition (First Embodiment)]

The method of producing a polythiol composition according to the first embodiment of the disclosure includes a generation step of generating a polythiol composition by decomposing a thiourethane resin with a decomposing agent represented by the following Formula (1) or (2).

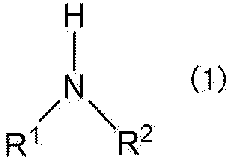

R³-OH (2)

In Formula (1), R¹ and R² each independently represent a hydrogen atom, an alkyl group having from 1 to 3 carbon atoms, or an amino group, excluding a case in which R¹ and R² are both an amino group.

In Formula (2), R³ represents an alkyl group having from 1 to 3 carbon atoms.

In the method of producing a polythiol composition according to the first embodiment, a thiourethane resin is decomposed by a decomposing agent represented by Formula (1) or (2), thereby generating a polythiol composition.

The method of producing a polythiol composition according to the first embodiment exhibits an excellent ability to remove a decomposing agent from a decomposition reaction system after the reaction of decomposing a thiourethane resin using the decomposing agent. For example, the decomposing agent can be easily removed from the reaction system after the decomposition reaction by volatilization, distillation, or the like.

The reason why such an effect is obtained is believed to be that the decomposing agent is a compound having a low boiling point.

Hereinafter, each step that may be included in the method of producing a polythiol composition according to the first embodiment will be described.

### <Generation Step>

The generation step is a step of decomposing a thiourethane resin using a decomposing agent represented by Formula (1) or (2) above (i.e., reacting a thiourethane resin with a decomposing agent), thereby generating a polythiol composition.

The decomposing agent represented by Formula (1) is an amine compound.

The decomposition reaction of decomposing a thiourethane resin using the decomposing agent represented by Formula (1) is an aminolysis reaction.

The decomposing agent represented by Formula (1) is an alcohol compound.

The decomposition reaction of decomposing a thiourethane resin using the decomposing agent represented by Formula (1) is an alcohololysis reaction.

### (Thiourethane Resin)

The thiourethane resin is a starting raw material in the generation step.

The thiourethane resin is not particularly limited. Examples thereof include thiourethane resins described in known literature such as JP-A No. S63-46213, JP-A No. H2-270859, JP-A No. H7-252207, WO 2008/047626, WO 2021/157701, and WO 2021/157702.

The thiourethane resin typically contains a polymer of an isocyanate compound and a polythiol composition. In other words, a thiourethane resin is usually produced using an isocyanate compound and a polythiol composition as raw materials.

### -Polythiol Composition as Raw Material for Thiourethane Resin-

In the disclosure, the polythiol composition (i.e., a polythiol composition as a raw material for the thiourethane resin and a polythiol composition described later as a target product in the method of producing a polythiol composition of the disclosure) means a composition containing at least one polythiol compound.

The polythiol compound is not particularly limited as long as it contains two or more thiol groups (also known as mercapto groups).

For the polythiol compound, the above-described publicly known literature regarding the thiourethane resin may be referred to, if appropriate.

The polythiol composition may contain components other than the polythiol compound as impurities.

The polythiol composition preferably contains at least one polythiol compound as a main component.

Here, the expression "polythiol composition contains at least one polythiol compound as a main component" means that the total content of at least one polythiol compound with respect to the total amount of the polythiol composition is 50% or more.

The total content of at least one polythiol compound with respect to the total amount of the polythiol composition is preferably 60% or more, more preferably 70% or more, and still more preferably 80% or more.

Similarly, in the disclosure, when a composition "contains...as a main component" a certain component (hereinafter referred to as "component X"), it means that the content of component X (in a case in which component X consists of two or more compounds, the total content of the two or more compounds) is 50% or more of the total amount of the composition.

The content of component X as a main component is preferably 60% or more, more preferably 70% or more, and still more preferably 80% or more with respect to the total amount of the composition.

The term "%" in the explanation of the expression "contains...as a main component" means the ratio (area%) of the total area of all peaks of component X (e.g., at least one polythiol compound) to the total area of all peaks of a composition (e.g., a polythiol composition) determined by high-performance liquid chromatography.

Hereinafter, the polythiol compound contained in the polythiol composition is also referred to as a "polythiol component."

It is preferable that the polythiol composition as a raw material for the thiourethane resin contains at least one (hereinafter also referred to as "polythiol component A") selected from the group consisting of 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, pentaerythritol tetrakis(2-mercaptoacetate), pentaerythritol tetrakis(3-mercaptopropionate), 2,5-dimercaptomethyl-1,4-dithiane, bis(2-mercaptoethyl)sulfide, and diethylene glycol bis(3-mercaptopropionate).

It is more preferable that the polythiol composition contains a polythiol component A as a main component.

In this case, the polythiol composition may contain at least one component other than the polythiol component A (e.g., another polythiol compound, a component other than a polythiol compound, or the like).

Examples of another polythiol compound include methanedithiol, 1,2-ethanedithiol, 1,2,3-propanetrithiol, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl)sulfide, 2,5-dimercapto-1,4-dithiane, 2,5-dimercaptomethyl-2,5-dimethyl-1,4-dithiane, 1,1,3,3-tetrakis(mercaptomethylthio)propane, 1,1,2,2-tetrakis(mercaptomethylthio)ethane, and 4,6-bis(mercaptomethylthio)-1,3-dithiane.

Examples of a more specific aspect of a polythiol composition as a raw material for the thiourethane resin include:
an aspect in which 4-mercaptomethyl-1,8-dimercapto-3,6-dithiaoctane (hereinafter also referred to as "polythiol component A1") is contained as a main component;
an aspect in which 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane (hereinafter, these three compounds are collectively referred to as "polythiol component A2") are contained as main components;
an aspect in which pentaerythritol tetrakis(3-mercaptopropionate) (hereinafter also referred to as "polythiol component A3") is contained as a main component;
an aspect in which polythiol components A1 and A3 are contained as main components; and
an aspect in which polythiol components A2 and A3 are contained as main components.

The polythiol composition of each embodiment may contain at least one component other than the main component (e.g., another polythiol compound, a component other than a polythiol compound, or the like).

### -Isocyanate Compound as Raw Material for Thiourethane Resin-

The isocyanate compound as a raw material for the thiourethane resin may be of only one kind or of two or more kinds.

Examples of the isocyanate compound as a raw material for the thiourethane resin include the known isocyanate compounds described in the above-described known literature.

The isocyanate compound as a raw material for the thiourethane resin contains preferably a polyisocyanate compound containing two or more isocyanate groups.

The isocyanate compound as a raw material for the thiourethane resin contains more preferably a diisocyanate compound containing two isocyanato groups, still more preferably at least one selected from the group consisting of pentamethylene diisocyanate, hexamethylene diisocyanate, m-xylylene diisocyanate, p-xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate (hereinafter also referred to as "isocyanate component N"), and even still more preferably an isocyanate component N as a main component.

The isocyanate compound as a raw material for the thiourethane resin contains yet still more preferably at least one selected from the group consisting of m-xylylene diisocyanate, 2,5-bis(isocyanatomethyl)bicyclo-[2.2.1]-heptane, 2,6-bis(isocyanatomethyl)bicyclo-[2.2.1]-heptane, isophorone diisocyanate, bis(isocyanatomethyl)cyclohexane, and bis(isocyanatocyclohexyl)methane (hereinafter also referred to as "isocyanate component N1"), and yet still more preferably an isocyanate component N1 as a main component from the viewpoint of the performance of the thiourethane resin[e.g., optical properties (e.g., refractive index and/or Abbe number), heat resistance, specific gravity d, and the like].

### -Other components-

The thiourethane resin may contain other components in addition to the polymer of at least one isocyanate compound and a polythiol composition.

For other components that can be contained in the thiourethane resin, the components that can be contained in the polymerizable composition described below can be referred to, if appropriate.

### -Recovered Thiourethane Resin-

It is preferable that the thiourethane resin is recovered during at least one of a process of manufacturing eyeglass lenses, a process of manufacturing eyeglasses, or a process of disposing of eyeglasses. According to this aspect, recycling of a thiourethane resin, which is a material for eyeglass lenses, can be realized.

Here, the process of manufacturing eyeglass lenses means a process of producing a resin by generating a monomer, which is a resin raw material, and performing cast polymerization, and/or milling a resin formed body, thereby obtaining eyeglass lenses.

The process of manufacturing eyeglasses means a process of manufacturing eyeglasses by combining eyeglass lenses with other members such as eyeglass frames.

The process of disposing of eyeglasses means a process of disposing of eyeglasses that have been manufactured but are no longer needed, used eyeglasses, and the like.

In either process, a thiourethane resin, a material used in eyeglass lenses, can be generated as waste.

In this aspect, a thiourethane resin generated in at least one of these processes is used as a starting material, and this thiourethane resin and a decomposing agent are reacted, thereby obtaining a polythiol composition as a decomposition product as of the thiourethane resin.

### -Processing Swarf Containing Thiourethane Resin-

In the generation step, a processing swarf containing thiourethane resin and a decomposing agent may be brought into contact, thereby reacting the thiourethane resin in processing swarf and the decomposing agent. This makes it possible to further improve the reaction efficiency between the thiourethane resin and the decomposing agent.

The form of the processing swarf containing the thiourethane resin is not particularly limited, and it may be in the form of powder or lumps.

The method of bringing the processing swarf containing a thiourethane resin and an amine compound into contact with each other is not particularly limited, and examples thereof include a method in which the processing swarf and the decomposing agent (and a reaction solvent, if necessary) are placed in a reaction vessel and stirred.

The processing swarf containing a thiourethane resin is preferably milling swarf (including the concept of polishing swarf, the same applies below) of a formed body containing a thiourethane resin and/or the milling swarf that has been sieved (i.e., milling swarf that has passed through a sieve).

The milling swarf of a formed body containing a thiourethane resin is generated, for example, when a formed body containing a thiourethane resin is cut so as to produce an optical material (e.g., lenses).

### -Resin Mixture Containing Thiourethane Resin-

The generation step may be a step of generating a polythiol composition by bringing a resin mixture containing a thiourethane resin and a decomposing agent into contact with each other, thereby reacting the thiourethane resin in the resin mixture and the decomposing agent.

The resin mixture containing a thiourethane resin further contains components other than a thiourethane resin.

Examples of components other than a thiourethane resin include resins other a thiourethane resin and inorganic materials for lens production (e.g., glass).

The resin other than a thiourethane resin is not particularly limited.

For example, the scope including the resin mixture containing a thiourethane resin and the resin other than a thiourethane resin encompasses:
a hybrid material of a thiourethane resin and a urethane resin, which is produced by adding a polyol compound to a raw material when producing a thiourethane resin;
a hybrid material of a thiourethane resin and a urea resin, which is produced by adding a polyamine compound to a raw material when producing a thiourethane resin;
and the like.

Examples of the resin other than a thiourethane resin also include:
a polyolefin film for protecting the surface of a resin formed body used in manufacturing eyeglass lenses;
a hard coat or primer coat for protecting the surface of a resin formed body for producing eyeglass lenses; an abrasive for use in polishing a resin formed body for producing eyeglass lenses;
a resin material for fixing a resin formed body when milling the resin formed body for producing eyeglass lenses;
tape or tape glue used for fixing a glass mold used when making a resin formed body for manufacturing eyeglass lenses;

The resin mixture preferably contains, as a resin other than a thiourethane resin, at least one resin selected from the group consisting of a polycarbonate resin, a polyallyl carbonate resin, an acrylic resin, a urethane resin, and an episulfide resin.

Like a thiourethane resin, these resins can also be used as materials for eyeglass lenses.

It is preferable that the resin mixture containing a thiourethane resin is recovered during at least one of a process of manufacturing eyeglass lenses, a process of manufacturing eyeglasses, or a process of disposing of eyeglasses.

The process of manufacturing eyeglass lenses, the process of manufacturing eyeglasses, and the process of disposing of eyeglasses have been described above.

The resin mixture containing a thiourethane resin preferably contains milling swarf containing a thiourethane resin.

### (Decomposing Agent Represented by Formula (1) or (2))

The decomposing agent used in this step is a decomposing agent represented by Formula (1) or (2) above.

Examples of the decomposing agent represented by Formula (1) include ammonia, monomethylamine, monoethylamine, monopropylamine, dimethylamine, diethylamine, dipropylamine, and hydrazine. Of these, ammonia, monomethylamine, and diethylamine are preferable.

Examples of the decomposing agent represented by Formula (2) include methanol, ethanol, and propanol. Of these, methanol or ethanol is preferable, and methanol is more preferable.

### (Charged Amount of Decomposing Agent)

In the generation step, the charged mass ratio of the decomposing agent to the thiourethane resin (i.e., charged mass ratio[decomposing agent/thiourethane resin]) can be adjusted, if appropriate, but is preferably from 0.10 to less than 2.0.

In a case in which the charged mass ratio[decomposing agent/thiourethane resin] is 0.10 or more, the generation of a polythiol composition is further promoted.

In a case in which the charged mass ratio[decomposing agent/thiourethane resin] is less than 2.0, it is possible to further suppress the decomposing agent from remaining in the reaction mixture.

The charged mass ratio[decomposing agent/thiourethane resin] is preferably from 0.10 to less than 1.0, more preferably from 0.15 to 0.95, and still more preferably from 0.20 to 0.90.

The millimole value of the decomposing agent charged with respect to 1 g of the thiourethane resin is preferably from 1.0 mmol/g to 50.0 mmol/g, more preferably from 2.0 mmol/g to 30.0 mmol/g, still more preferably from 3.0 mmol/g to 20.0 mmol/g.

The charged equivalent of the decomposing agent with respect to the thiourethane resin (charged equivalent[decomposing agent/thiourethane resin]) is preferably from 1.0 to 15.0, more preferably from 1.0 to 10.0, and still more preferably from more than 1.0 to 10.0.

In a case in which the charged equivalent[decomposing agent/thiourethane resin] is 1.0 or more, the generation of the polythiol composition is further promoted.

In a case in which the charged equivalent[decomposing agent/thiourethane resin] is 15.0 or less, it is possible to further suppress the decomposing agent from remaining in the reaction mixture.

### (Tertiary Amine Compound as Decomposition Aid)

In the case of using a decomposing agent represented by Formula (2) above as the decomposing agent, in the generation step, it is preferable to decompose a thiourethane resin using a decomposing agent represented by Formula (2) in the presence of a tertiary amine compound as a decomposition aid, thereby generating a polythiol composition.

The tertiary amine compound as a decomposition aid is not particularly limited as long as it is a tertiary amine compound.

The tertiary amine compound as a decomposition aid may be a chain amine compound or a cyclic amine compound.

The molecular weight of the tertiary amine compound as a decomposition aid is preferably 1000 or less, more preferably 500 or less, still more preferably 300 or less, and even still more preferably 200 or less.

The lower limit of the molecular weight of the tertiary amine compound as a decomposition aid is 59 or more and more preferably 70 or more.

The tertiary amine compound as a decomposition aid is not particularly limited as long as it is a tertiary amine compound.

Examples of the tertiary amine compound as a decomposition aid include: preferably N,N-dimethylethanolamine, N,N-dimethylaminopropanol, N,N-diethylaminoethanol, N-methyldiethanolamine, diisopropylethylamine, triethylamine, triisopropylamine, triisobutylamine, N,N-dimethylcyclohexylamine, N-methylmorpholine, N-ethylmorpholine, dimethylpiperazine, 1-ethylpiperidine, 4-(2-hydroxyethyl)morpholine, 1,4-diazabicyclo[2,2,2]octane, diazabicyclononene, or diazabicycloundecene; more preferably N,N-dimethylethanolamine, N,N-diethylaminoethanol, N-methyldiethanolamine, diisopropylethylamine, triisopropylamine, triisobutylamine, N,N-dimethylcyclohexylamine, N-methylmorpholine, N-ethylmorpholine, dimethylpiperazine, 1-ethylpiperidine, 1,4-diazabicyclo[2,2,2]octane, diazabicyclononene, or diazabicycloundecene; and still more preferably N,N-dimethylethanolamine, diisopropylethylamine, N,N-dimethylcyclohexylamine, N-ethylmorpholine, or 1,4-diazabicyclo[2,2,2]octane.

The charged mass ratio of the tertiary amine compound as a decomposition aid to the decomposing agent represented by Formula (2) as the decomposing agent (i.e., the charged mass ratio[decomposition aid/decomposing agent]) can be adjusted, if appropriate, but is preferably from 0.001 to 2.00.

The charged mass ratio[decomposition aid/decomposing agent] is preferably from 0.002 to 1.50 and more preferably from 0.004 to 1.20.

The charged mole ratio of the tertiary amine compound as a decomposition aid to the decomposing agent represented by Formula (2) as the decomposing agent (i.e., the charged mole ratio[decomposition aid/decomposing agent]) can be adjusted, if appropriate, but is preferably from 0.001 to 3.00.

The charged mole ratio[decomposition aid/decomposing agent] is preferably from 0.002 to 2.50, more preferably from 0.003 to 2.00, still more preferably from 0.004 to 1.50, and even still more preferably from 0.004 to 1.00.

### (Reaction Solvent)

In the generation step, it is preferable to react with the thiourethane resin with the decomposing agent represented by Formula (1) or (2) in the presence of a reaction solvent.

The reaction solvent is preferably an organic solvent, more preferably a hydrocarbon compound having from 5 to 12 carbon atoms (preferably from 6 to 10 and more preferably from 7 to 9).

The hydrocarbon compound is preferably hexane, heptane, octane, nonane, decane, xylene, mesitylene, or toluene, more preferably heptane, octane, nonane, xylene, mesitylene, or toluene, and particularly preferably xylene or toluene.

The reaction solvent may be of only one kind or of two or more kinds.

### (Reaction Temperature)

The reaction temperature for reacting the thiourethane resin and the decomposing agent represented by Formula (1) or (2) in the generation step can be adjusted, if appropriate.

In the generation step, it is preferable to bring the thiourethane resin and the decomposing agent represented by Formula (1) or (2) into contact with each other at temperature conditions of from 50°C to 200°C (more preferably from 60°C to 180°C and still more preferably from 70°C to 150°C) (i.e., reaction temperatures).

In a case in which the reaction temperature is from 50°C to 200°C, it is possible to improve the purity of a polythiol component as a main component in a polythiol composition as a target product (i.e., the content of the main component with respect to the entire amount of the polythiol composition).

### (Reaction Time)

The time for a reaction between the thiourethane resin and the decomposing agent represented by Formula (1) or (2) the in generation step can be adjusted, if appropriate. However, it is preferably from 0.1 hours to 20 hours, more preferably from 0.5 hours to 16 hours, and still more preferably from 1 hour to 10 hours.

### (Reaction Pressure)

It is preferable that the generation step includes reacting a thiourethane resin with a decomposing agent represented by Formula (1) or (2) under a pressure higher than atmospheric pressure. This further suppresses the volatilization of the decomposing agent represented by Formula (1) or (2).

In this case, the method includes reacting the thiourethane resin with the decomposing agent represented by Formula (1) or (2) under a pressure that is higher than atmospheric pressure by preferably from 0.01 MPa or more (more preferably from 0.01 MPa to 2.0 MPa and still more preferably from 0.02 MPa to 1.0 MPa).

### (Polythiol Composition as Target Product)

The polythiol composition as a target product in the method of producing a polythiol composition according to the first embodiment and preferred aspects thereof are the same as the polythiol composition that is a raw material for the thiourethane resin described above and preferred aspects thereof.

The polythiol composition as the target product and the polythiol composition that is the raw material for the thiourethane resin as the starting material do not need to be completely identical.

Note that from the viewpoint of performance of a thiourethane resin produced with the polythiol composition as the target product, it is preferable that the type of polythiol component as the main component in the target polythiol composition is the same as the type of polythiol component as the main component in the polythiol composition that is the raw material for the thiourethane resin as the starting material. In this case, for example, an optical material B (an optical material containing a thiourethane resin) having performance comparable to that of an optical material A can be produced using milling swarf (thiourethane resin) generated during the production of the optical material A as a raw material.

The polythiol composition as the target product may have the same polythiol component that is the main component as the polythiol composition that is the raw material of the thiourethane resin as the starting material, and may have a reduced content of impurities.

In a case in which the content of impurities in the polythiol composition as the target product is reduced, thickening of the polythiol composition is suppressed, and the polythiol composition can have the advantage of having a long pot life.

There are no particular limitations on the applications of the polythiol composition as the target product.

The polythiol composition as the target product can be used, for example, in producing a thiourethane resin.

Specific applications of the polythiol composition as the target product include polythiol compositions for producing optical materials (e.g., eyeglass lenses).

### (Polyurea Compound or Polycarbamate Compound)

In the generation step in the first embodiment, not only a polythiol composition but also a polyurea compound and a polycarbamate compound can be generated by decomposing the thiourethane resin using a decomposing agent.

Specifically, in the case of using the decomposing agent represented by Formula (1) as such a decomposing agent, the decomposition of the thiourethane resin can generate a polythiol composition and a polyurea compound. The generation step of the method of producing a polythiol composition in this case may also correspond to the first step of the method of producing a polyamine compound according to the second embodiment described below.

In the case of using the decomposing agent represented by Formula (2) as such a decomposing agent, the decomposition of the thiourethane resin can generate a polythiol composition and a polycarbamate compound.

The generation step of the method of producing a polythiol composition in this case may also correspond to Step X1 of the method of producing a polyamine compound according to the third embodiment described below.

### (Reaction Mixture Containing Polythiol Composition)

The generation step may be a step of decomposing the thiourethane resin with a decomposing agent to generate a polythiol composition and obtain a reaction mixture containing the polythiol composition as the target product.

Examples of components other than the polythiol composition in the reaction mixture include the above-described reaction solvent, residues of raw materials (the thiourethane resin and/or the decomposing agent), impurities contained in the raw materials, the above-described polyurea compound, and the above-described polycarbamate compound.

### <Separation Step>

The method of producing a polythiol composition according to the first embodiment may include a separation step of separating a polythiol composition from the reaction mixture containing a polythiol composition generated in the generation step.

The separation method in the separation step is not particularly limited, and any known method can be applied.

Examples of the separation method in the separation step include filtration, decantation, extraction, distillation, drying (including drying under reduced pressure), and purification (e.g., column chromatography). As the separation method, a plurality of methods may be used in combination.

The separation step preferably includes obtaining a filtrate containing the polythiol composition by filtering the reaction mixture containing the polythiol composition obtained in the reaction step.

According to this aspect, it is easier to remove a solid contained in the reaction mixture (e.g., a solid containing by-products).

One more preferred aspect in the aspect in which the separation step includes obtaining a filtrate containing a polythiol composition is an aspect (hereinafter referred to as "separation aspect A") in which
the separation step includes:
obtaining a filtrate containing a polythiol composition by filtering a reaction mixture containing the polythiol composition;
acid-washing the filtrate containing the polythiol composition; and
separating the polythiol composition from the acid-washed filtrate.

According to the separation aspect A, as acid washing makes it easy to remove an alkaline component (e.g., a residual amine compound) from the filtrate, a polythiol composition having a higher purity of a polythiol component as a main component can be obtained.

In separation aspect A, aqueous washing may be added after acid washing, thereby separating a polythiol composition from a filtrate after aqueous washing.

In separation aspect A, examples of the acid used for the acid washing include hydrochloric acid, carbonic acid, nitric acid, sulfuric acid, acetic acid, formic acid, and oxalic acid.

One more preferred aspect in the aspect in which the separation step includes obtaining a filtrate containing a polythiol composition is an aspect (hereinafter referred to as "separation aspect B") in which
the separation step includes:
obtaining a filtrate containing a polythiol composition by filtering a reaction mixture containing the polythiol composition;
adding a base containing an alkali metal to the filtrate containing the polythiol composition, and then adding water to perform, thereby obtaining a water extract containing an alkali metal salt of the polythiol composition;
adding an acid to the water extract containing the alkali metal salt of the polythiol composition to obtain an aqueous liquid containing the polythiol composition;
adding a hydrocarbon compound having from 5 to 12 carbon atoms as an extraction solvent to an aqueous liquid containing a polythiol composition and performing extraction to obtain an extract containing the polythiol composition; and
separating the polythiol composition from the extract containing the polythiol composition.

In separation aspect B, first, the polythiol composition in the filtrate containing the polythiol composition is converted to an alkali metal salt, and then extraction is performed with water, thereby obtaining a water extract containing the alkali metal salt of the polythiol composition. Then, an acid is added to the mixture to convert the alkali metal salt of the polythiol composition back into the polythiol composition. The polythiol composition is extracted from the resulting aqueous liquid containing the polythiol composition with the extraction solvent, thereby obtaining an extract containing the polythiol composition. The polythiol composition is separated from the resulting extract containing the polythiol composition.

According to separation aspect B, even in a case in which the filtrate containing the polythiol composition contains a large amount of components other than the polythiol composition, a polythiol composition having a high purity of a polythiol component as a main component can be obtained.

In separation aspect B, the alkali metal in the base containing an alkali metal is preferably sodium, potassium, or lithium, and more preferably sodium or potassium.

Examples of the base containing an alkali metal include sodium methoxide, sodium ethoxide, sodium propoxide, sodium hydroxide, potassium hydroxide, and lithium hydroxide.

The base containing an alkali metal can be added to the filtrate in the form of an alcohol solution (methanol solution, ethanol solution, or the like) if necessary.

In separation aspect B, examples of the acid that can be added to the aqueous extract containing the alkali metal salt of the polythiol composition include hydrochloric acid, carbonic acid, nitric acid, sulfuric acid, acetic acid, formic acid, and oxalic acid.

In separation aspect B, the hydrocarbon compound used as the extraction solvent may be of only one kind or of two or more kinds.

In separation aspect B, preferred aspects of the hydrocarbon compound as the extraction solvent are the same as preferred aspects of the hydrocarbon compound as the reaction solvent described above.

Note that the reaction solvent and the extraction solvent may be the same or different.

One more preferred aspect in the aspect in which the separation step includes obtaining a filtrate containing a polythiol composition is an aspect (hereinafter referred to as "separation aspect C") in which
the separation step includes:
separating a reaction mixture containing a polythiol composition by decantation to obtain a residue containing the polythiol composition;
adding a base containing an alkali metal to the residue containing the polythiol composition, and then adding water to perform, thereby obtaining a water extract containing an alkali metal salt of the polythiol composition;
adding an acid to the water extract containing the alkali metal salt of the polythiol composition to obtain an aqueous liquid containing the polythiol composition;
adding a hydrocarbon compound having from 5 to 12 carbon atoms as an extraction solvent to an aqueous liquid containing a polythiol composition and performing extraction to obtain an extract containing the polythiol composition; and
separating the polythiol composition from the extract containing the polythiol composition.

The separation aspect C is the same as the separation step B, except that the residue containing the polythiol composition is obtained by a decantation method.

### <Classification Step>

The method of producing a polythiol composition according to the first embodiment may further include a classification step of classifying milling swarf containing a thiourethane resin before the reaction step. In this case, the classified milling swarf is reacted with a decomposing agent represented by Formula (1) or (2) in the reaction step.

In a case in which the method of producing a polythiol composition according to the first embodiment includes the classification step, as milling swarf consisting of particles having a small particle size (i.e., average particle size) is contacted with a decomposing agent represented by Formula (1) or (2) in the reaction step, the efficiency of the reaction between the thiourethane resin and the decomposing agent represented by Formula (1) or (2) can be further improved.

The average particle size in the disclosure may be, for example, a number average particle size.

The particle size may be, for example, a circle equivalent diameter.

Examples of the classification method include sieving and centrifugation.

For an aspect in which sieving is performed as classification, the sieving step described below can be referred to.

### <Sieving Step>

The method of producing a polythiol composition according to the first embodiment may further include a sieving step of sieving milling swarf containing a thiourethane resin before the generation step. In this case, in the generation step, milling swarf that has passed through a sieve is brought into contact with a decomposing agent represented by Formula (1) or (2), thereby reacting a thiourethane resin in the milling swarf that has passed through a sieve with the decomposing agent represented by Formula (1) or (2).

In a case in which the method of producing a polythiol composition according to the first embodiment includes the classification step, as milling swarf consisting of particles having a small particle size is contacted with a decomposing agent represented by Formula (1) or (2) in the generation step, the efficiency of the reaction between the thiourethane resin and the decomposing agent represented by Formula (1) or (2) can be further improved.

The above sieve is not particularly limited.

The nominal mesh size of the sieve as specified in JIS Z-8801-1:2019 is, for example, from 0.1 mm to 20 mm, preferably from 0.1 mm to 10 mm, more preferably from 0.1 mm to 5 mm, still more preferably from 0.1 mm to 2 mm, even still more preferably from 0.3 mm to 2 mm, and yet still more preferably from 0.5 mm to 1.5 mm.

### <Washing Step>

The method of producing a polythiol composition according to the first embodiment may further include a washing step of washing a thiourethane resin with a hydrocarbon compound having from 5 to 12 carbon atoms as a washing solvent before the generation step. In this case, the thiourethane resin washed in the washing step is reacted with a decomposing agent represented by Formula (1) or (2) in the reaction step. This allows a polythiol composition having a higher purity of a polythiol component as a main component to be obtained.

In particular, in a case in which milling swarf containing a thiourethane resin is used as a starting material in the method of producing a polythiol composition according to the first embodiment, an oil from a milling machine which adheres to the milling swarf can be removed in the above-described washing step, allowing a polythiol composition having a higher purity of a polythiol component as a main component to be obtained.

The hydrocarbon compound as a washing solvent may be of only one kind or of two or more kinds.

Preferred aspects of the hydrocarbon compound as the washing solvent are the same as preferred aspects of the hydrocarbon compound as the reaction solvent described above.

Note that the reaction solvent and the washing solvent may be the same or different.

The washing method in the washing step is not particularly limited. A known method can be applied, such as a method in which the above-described washing solvent is added to milling swarf containing a thiourethane resin (e.g., thiourethane resin swarf) and mixed.

In a case in which the method of producing a polythiol composition according to the first embodiment includes the sieving step and the washing step described above, it is preferable to carry out the sieving step and the washing step in such an order. In this case, since there is no need to wash away the milling swarf that has not passed through the sieve, the amount of washing solvent used can be further reduced.

### [Method of Producing Polymerizable Composition (First Embodiment)]

The method of producing a polymerizable composition according to the first embodiment of the disclosure includes:
a step of producing a polythiol composition by the above-described method of producing a polythiol composition according to the first embodiment; and
a step of mixing at least the polythiol composition with a polyisocyanate compound, thereby obtaining a polymerizable composition containing the polythiol composition and the polyisocyanate compound.

The method of producing a polymerizable composition according to the first embodiment may include other steps, if necessary.

In the method of producing a polymerizable composition according to the first embodiment, a polythiol composition is produced using a thiourethane resin (e.g., a thiourethane resin in milling swarf of a thiourethane resin formed body) as a starting material in the step of producing a polythiol composition, and a polymerizable composition containing the polythiol composition produced above and a polyisocyanate compound is produced in the step of obtaining a polymerizable composition.

The obtained polymerizable composition can be reused for producing a thiourethane resin.

In this manner, effective utilization (i.e., recycling) of materials (i.e., the thiourethane resin and the polythiol composition that is the raw material thereof) is realized in the method of producing a polymerizable composition according to the first embodiment.

In addition, according to the method of producing a polythiol composition according to the first embodiment, compared with known methods (e.g., a method of obtaining a polythiol composition by reacting a thiourethane resin with sodium hydroxide), a polythiol composition having a high purity of a polythiol component as a main component can be obtained.

In the method of producing a polymerizable composition according to the first embodiment, such a polythiol composition is used. Therefore, a resin excellent in various properties (e.g., optical properties (e.g., refractive index and/or Abbe number), heat resistance, and specific gravity d) can be produced with the polymerizable composition obtained by the method of producing a polymerizable composition according to the first embodiment.

Accordingly, the polymerizable composition obtained by the method of producing a polymerizable composition according to the first embodiment is particularly suitable as a composition for producing a thiourethane resin for optical materials.

### <Step of Producing Polythiol Composition>

For the step of producing a polythiol composition in the method of producing a polymerizable composition according to the first embodiment, the above-described the method of producing a polythiol composition according to the first embodiment can be referred to, if appropriate.

### <Step of Obtaining Polymerizable Composition>

In the step of obtaining a polymerizable composition, by mixing at least the above-described polythiol composition with a polyisocyanate compound, a polymerizable composition containing the polythiol composition and the polyisocyanate compound are obtained.

Preferred aspects of the polyisocyanate compound used in the step of obtaining a polymerizable composition are the same as preferred aspects of the "isocyanate compound as a raw material for a thiourethane resin" explained in the section "Method of Producing Polythiol Composition."

In the step of obtaining a polymerizable composition, the mixing ratio of the polythiol composition and the polyisocyanate compound is not particularly limited.

In the step of obtaining a polymerizable composition, the ratio of the charged mass of the polythiol composition to the charged mass of the polyisocyanate compound (i.e., charged mass[polythiol composition/polyisocyanate compound]) is preferably from 0.10 to 10.0, more preferably from 0.20 to 5.00, still more preferably from 0.50 to 1.50, and even sill more preferably from 0.70 to 1.30.

It is further preferable that the molar ratio of the mercapto group of the polythiol compound to the isocyanato group of the polyisocyanate compound contained in the polythiol composition (mercapto group/isocyanato group) is preferably from 0.5 to 3.0, more preferably from 0.6 to 2.0, and more preferably from 0.8 to 1.3.

In the step of obtaining a polymerizable composition, the total charged mass of the polythiol composition and the polyisocyanate compound is not particularly limited. However, it is preferably 60% by mass or more, more preferably 80% by mass or more, and still more preferably 90% by mass or more with respect to the total amount of the polymerizable composition to be produced.

The step of obtaining a polymerizable composition may also be a step of mixing the polythiol composition with a polyisocyanate composition containing the polyisocyanate compound, thereby obtaining a polymerizable composition containing the polythiol composition and the polyisocyanate composition,

Here, the polyisocyanate composition means a composition containing at least one polyisocyanate compound.

The polyisocyanate composition may contain components other than the polyisocyanate compound as impurities.

The polyisocyanate composition preferably contains at least one polyisocyanate compound as a main component.

The meaning of the expression "contains...as a main component" is as described above.

The polyisocyanate composition preferably contains a xylylene diisocyanate.

Hereinafter, the polyisocyanate composition containing a xylylene diisocyanate is also referred to as an XDI composition.

The XDI composition preferably contains a xylylene diisocyanate as a main component.

The XDI composition preferably contains at least one selected from the group consisting of the following compound (N1), the following compound (N2), and the following compound (N3).

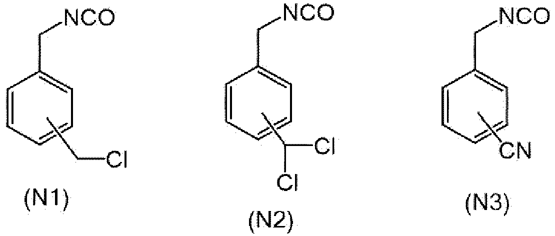

Hereinafter, preferred embodiments of the XDI composition will be described from the viewpoint of achieving excellent stability of the polyisocyanate composition and transparency of a resin formed using the polyisocyanate composition.

In a case in which the XDI composition contains the compound (N1), the peak area of the compound (N1) measured by gas chromatography under the following GC conditions 1 corresponds to preferably 0.20 ppm or more with respect to a peak area of 1 of the xylylene diisocyanate.

### -GC Conditions 1-

Filler: DB-1 (film thickness) 1.5 µm
Column: inner diameter of 0.53 mm × length of 60 m (Agilent Technologies, Inc.)
Oven temperature: Increased from 130°C to 220°C at 3°C/min, and after reaching 220°C, increased to 300°C at 10°C/min.
Split ratio: Pulsed splitless method
Inlet temperature: 280°C
Detector temperature: 300°C
Carrier gas: N₂ 158kPa, H₂ 55kPa, Air 45kPa (constant pressure control)
Solvent: Chloroform
Sample concentration: 2.0% by mass in chloroform
Injection volume: 2 µL
Detection method: FID

The peak area of the compound (N1) corresponds to more preferably 5.0 ppm or more, still more preferably 50 ppm or more, and even still more preferably 100 ppm or more with respect to a peak area of 1 of the xylylene diisocyanate.

The peak area of the compound (N1) corresponds to preferably 4000 ppm or less, more preferably 3000 ppm or less, still more preferably 2000 ppm or less, even still more preferably 1500 ppm or less, and yet still more preferably 1000 ppm or less with respect to a peak area of 1 of the xylylene diisocyanate.

The peak area of the compound (N1) can be measured in accordance with the method described in paragraph 0377 of Japanese Patent No. 6,373,536.

In a case in which the XDI composition contains the compound (N2), the peak area of the compound (N2) measured by gas chromatography under the following GC conditions 2 corresponds to preferably 0.05 ppm or more with respect to a peak area of 1 of the xylylene diisocyanate.

### -GC Conditions 2-

Column: HP-50+, inner diameter of 0.25 mm × length of 30 m × film thickness of 0.25 µm (manufactured by Hewlett Packard Company)
Oven temperature: Increased from 50°C to 280°C at a rate of 10°C/min, and held at 280°C for 6 min.
Split ratio: Pulsed splitless method
Inlet temperature: 200°C
Detector temperature: 280°C
Carrier gas: He
Carrier gas flow rate: 1.0 mL/min (constant flow rate control)
Sample concentration: 1.0% by mass in a dichloromethane solution
Injection volume: 1.0 µL
Detection method: SIM (monitoring ions: m/z 180, 215) (xylylene diisocyanate (XDI) content)

The peak area of the compound (N2) corresponds to more preferably 0.1 ppm or more, still more preferably 0.3 ppm or more, and even still more preferably 0.6 ppm or more with respect to a peak area of 1 of the xylylene diisocyanate.

The peak area of the compound (N2) corresponds to preferably 200 ppm or less, more preferably 150 ppm or less, still more preferably 100 ppm or less, even still more preferably 80 ppm or less, yet still more preferably 70 ppm or less, and yet still more preferably 60 ppm or less with respect to a peak area of 1 of the xylylene diisocyanate.

The peak area of the compound (N2) can be measured in accordance with the method described in paragraphs 0375 and 0376 of Japanese Patent No. 6,373,536.

In a case in which the XDI composition contains the compound (N3), the peak area of the compound (N3) measured by gas chromatography under the above GC conditions 1 corresponds to preferably 0.10 ppm or more with respect to a peak area of 1 of the xylylene diisocyanate.

The peak area of the compound (N3) corresponds to more preferably 0.1 ppm or more, still more preferably 3.0 ppm or more, and even still more preferably 5.0 ppm or more with respect to a peak area of 1 of the xylylene diisocyanate.

The peak area of the compound (N3) corresponds to preferably 1000 ppm or less, more preferably 500 ppm or less, still more preferably 300 ppm or less, even still more preferably 100 ppm or less, and yet still more preferably 75 ppm or less with respect to a peak area of 1 of the xylylene diisocyanate.

The peak area of the compound (N3) can be measured in accordance with the method described in paragraph 0377 of Japanese Patent No. 6,373,536.

The acid content of the XDI composition is preferably 3000 ppm or less, more preferably 2000 ppm or less, still more preferably 1000 ppm or less, even still more preferably 100 ppm or less, yet still more preferably 50 ppm or less, yet still more preferably 30 ppm or less, and yet still more preferably less than 15 ppm.

The lower limit of the acid content of the XDI composition is not particularly limited, but the lower limit is, for example, 1 ppm.

The acid content of the XDI composition can be measured according to the method described in paragraph 0091 of WO 2021/256417.

The XDI composition may also contain a stabilizer.

In the step of obtaining a polymerizable composition, at least the polythiol composition and the polyisocyanate compound described above are mixed. If necessary, the polythiol composition, the polyisocyanate compound, and other components may be mixed as well.

In addition, in the step of obtaining a polymerizable composition, at least the polythiol composition and the polyisocyanate compound may be mixed, and then other components may be mixed into the mixture.

Examples of these other components include a polymerization catalyst, an internal mold release agent, a resin modifier, a chain extender, a crosslinking agent, a radical scavenger, a light stabilizer, an ultraviolet absorber, an antioxidant, an oil-soluble dye, a filler, an adhesion improver, an antibacterial agent, an antistatic agent, a dye, a fluorescent brightener, a fluorescent pigment, and an inorganic pigment.

Examples of the polymerization catalyst include a tertiary amine compound, an inorganic or organic acid salt thereof, a metal compound, a quaternary ammonium salt, and an organic sulfonic acid.

As the internal mold release agent, an acidic phosphoric acid ester can be used. Examples of the acidic phosphate ester include a monophosphate ester and a diphosphate ester, each of which may be used singly, or in mixture of two or more kinds thereof.

Examples of the resin modifier include an episulfide compound, an alcohol compound, an amine compound, an epoxy compound, an organic acid, an anhydride of an organic acid, and an olefin compound including a (meth)acrylate compound. Here, the (meth)acrylate compound means at least one of an acrylate compound or a methacrylate compound.

In the step of obtaining a polymerizable composition, the above-described components can be mixed by an ordinary method, and the mixing method is not particularly limited.

### [Method of Producing Resin (First Embodiment)]

The method of producing a resin according to the first embodiment of the disclosure includes:
a step of producing a polymerizable composition by the above-described method of producing a polymerizable composition according to the first embodiment; and
a step of obtaining a resin by curing the polymerizable composition.

The method of producing a resin according to the first embodiment may include other steps, if necessary.

The resin produced by the method of producing a resin according to the first embodiment and the resin according to the first embodiment described below are each a thiourethane resin. However, in the disclosure, they are simply referred to as "resin" to distinguish them from the thiourethane resin that is one of the starting materials for the polythiol composition.

In the step of obtaining a resin, a resin is obtained by curing the above-described polymerizable composition.

The polymerizable composition can be cured by polymerizing monomers in the polymerizable composition (specifically, a polythiol composition and a polyisocyanate compound; the same applies below). As a pretreatment for polymerization, the polymerizable composition may be subjected to treatments such as filtration and degassing.

The polymerization conditions (e.g., polymerization temperature, polymerization time, and the like) for polymerizing the monomers in the polymerizable composition are set, if appropriate, in consideration of the configuration of the composition, the type and amount of the monomers in the composition, the type and amount of the polymerization catalyst in the composition, and the properties of the mold in the case of using a mold described below.

The polymerization temperature may be, for example, from -50°C to 150°C or from 10°C to 150°C.

The polymerization time may be, for example, from 1 hour to 200 hours or from 1 hour to 80 hours.

In the step of obtaining a resin, the polymer obtained by polymerization of the monomers may be subjected to a treatment such as annealing, thereby obtaining the resin.

The annealing temperature may be from 50°C to 150°C, from 90°C to 140°C, from 100°C to 130°C, or the like.

### [Method of Producing Formed body (First Embodiment)]

The method of producing a formed body according to the first embodiment of the disclosure is a method of producing a formed body containing a resin, the method including:
a step of producing a polymerizable composition by the above-described method of producing a polymerizable composition according to the first embodiment; and
a step of obtaining a formed body containing a resin by curing the polymerizable composition.

The method of producing a formed body according to the first embodiment may include other steps, if necessary.

In the step of obtaining a formed body containing a resin, a formed body containing a resin is obtained by curing the above-described polymerizable composition.

For preferred conditions for curing the polymerizable composition, i.e., for polymerization of the monomers in the polymerizable composition, the section "METHOD OF Producing Resin" can be referred to, if appropriate.

### An example of the polymerization in this step is cast polymerization

In cast polymerization, first, the polymerizable composition is poured between a mold held by a gasket, tape, or the like. At this time, defoaming treatment, filtration treatment, or the like may be performed as necessary.

Next, the monomers in the polymerizable composition injected between molding molds is polymerized, thereby curing the composition between the molding molds to obtain a cured product. The cured product is then removed from the mold, thereby obtaining a formed body containing a resin.

The polymerization of the monomers may be carried out by heating the polymerizable composition. This heating can be carried out, for example, using a heating device equipped with a mechanism for heating the object to be heated in an oven, water, or the like.

### [Method of Producing Optical Material (First Embodiment), Method of Producing Lens (First Embodiment)]

The method of producing an optical material (e.g., lens) according to the first embodiment of the disclosure is a method of producing an optical material (e.g., lens) including a formed body containing a resin, the method including:
a step of producing a polymerizable composition by the above-described method of producing a polymerizable composition according to the first embodiment; and
a step of obtaining a formed body containing a resin by curing the polymerizable composition.

The method of producing an optical material (e.g., lens; the same applies below) according to the first embodiment may include other steps as necessary.

The method of producing an optical material according to the first embodiment is an application of the method of producing a formed body according to the first embodiment.

For example, in the method of producing a formed body according to the first embodiment, by selecting the shape of the molding mold used in the above-described cast polymerization, if appropriate, a formed body applicable to an optical material (e.g., lens) can be obtained.

Examples of the optical material include lenses (e.g., eyeglass lenses, camera lenses, polarized lenses) and light emitting diodes (LEDs).

The method of producing an optical material (e.g., lens) according to the first embodiment may include a step of forming a coating layer on one or both sides of a formed body containing a resin.

Specific examples of the coating layer include a primer layer, a hard coat layer, an anti-reflection layer, an anti-fogging coat layer, an anti-fouling layer, and a water-repellent layer.

Each of these coating layers may be formed individually, or a plurality of coating layers may be formed in a multi-layer structure. In the case of forming a coating layer on both sides, the same coating layer may be formed on each side, or different coating layers may be formed on the respective sides.

The components of the coating layer can be selected depending on the purpose, if appropriate.

Examples of components of the coating layer include a resin (e.g., urethane resin, epoxy resin, polyester resin, melamine resin, polyvinyl acetal resin, or the like), an infrared absorber, a light stabilizer, an antioxidant, a photochromic compound, a dye, a pigment, and an antistatic agent.

For details about eyeglass lenses and coating layers, the descriptions in publicly known literature such as WO 2017/047745 can be referred to, if appropriate.

### [Polymerizable Composition (First Embodiment)]

The polymerizable composition according to the first embodiment of the disclosure contains the polythiol composition and the polyisocyanate compound obtained by the above-described the method of producing a polythiol composition according to the first embodiment.

The polymerizable composition according to the first embodiment can be produced by the method of producing a polymerizable composition according to the first embodiment described above.

For preferred aspects of the polymerizable composition according to the first embodiment, the above-described method of producing a polymerizable composition according to the first embodiment can be referred to, if appropriate.

Note that the charged mass[polythiol composition/polyisocyanate compound] is read as the content mass ratio[polythiol composition/polyisocyanate compound], and the total charged mass of the polythiol composition and the polyisocyanate compound is read as the total content mass of the polythiol composition and the polyisocyanate compound.

### [Resin (First Embodiment), Formed body (First Embodiment), Optical Material (e.g., Lens) (First Embodiment)]

The resin according to the first embodiment of the disclosure is a cured product of the polymerizable composition according to the first embodiment of the disclosure described above.

The formed body according to the first embodiment of the disclosure is a formed body containing the resin according to the first embodiment described above.

The optical material (e.g., lens) according to the first embodiment is an optical material (e.g., lens) containing the resin according to the first embodiment described above.

The resin according to the first embodiment, the formed body according to the first embodiment, and the optical material (e.g., lens) according to first embodiment can be produced by the method of producing a resin according to the first embodiment, the method of producing a formed body according to the first embodiment, and the method of producing an optical material (e.g., lens) according to the first embodiment described above, respectively.

For preferred aspects of the resin according to the first embodiment, the formed body according to the first embodiment, and the optical material (e.g., lens) according to the first embodiment, the preferred aspects of the method of producing a resin according to the first embodiment, the method of producing a formed body according to the first embodiment, and the method of producing an optical material (e.g., lens) according to the first embodiment described above can be referred to.

### <Preferred Performance of Resin or Formed body>

From the viewpoint of heat resistance, the glass transition temperature Tg of the resin (or formed body) according to the first embodiment is preferably 70°C or more, more preferably 80°C or more, and still more preferably 85°C or more.

The glass transition temperature Tg may be 130°C or less, 120°C or less, or 110°C or less.

From the viewpoint of the application to optical materials, the refractive index (ne) of the resin (or formed body) according to the first embodiment is preferably 1.500 or more, more preferably 1.540 or more, and still more preferably 1.590 or more.

The upper limit of the refractive index (ne) is not particularly limited, but the upper limit is, for example, 1.750.

The Abbe number of the resin (or formed body) according to the first embodiment is preferably 28 or more, and more preferably 30 or more, from the viewpoint of the application to optical materials.

The upper limit of the Abbe number is not particularly limited, but the upper limit is, for example, 50, and preferably 45.

From the viewpoint of the application to optical materials, the specific gravity d of the resin (or formed body) according to the first embodiment is preferably 1.10 or more, and more preferably 1.20 or more.

The upper limit of the specific gravity d is not particularly limited, but the upper limit is, for example, 1.50, and preferably 1.40.

### <<Second Embodiment>>

### [Method of Producing Polyamine Compound (Second Embodiment)]

The method of producing a polyamine compound according to the second embodiment includes:
a first step of generating a polyurea compound by decomposing a thiourethane resin using a decomposing agent represented by the following Formula (1); and
a second step of generating a polyamine compound by decomposing the polyurea compound using a decomposing agent represented by the following Formula (3):

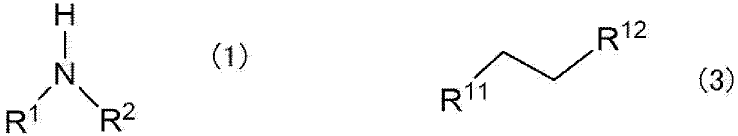

In Formula (1), R¹ and R² each independently represent a hydrogen atom, an alkyl group having from 1 to 3 carbon atoms, or an amino group , excluding a case in which R¹ and R² are both an amino group.

In Formula (3), R¹¹ and R¹² each independently represent a hydroxy group, a mercapto group, an amino group, or a monomethylamino group.

The method of producing a polyamine compound according to the second embodiment exhibits an excellent ability to remove a decomposing agent represented by Formula (1) from a decomposition reaction system after the decomposition reaction in the first step. For example, the decomposing agent represented by Formula (1) can be easily removed from the reaction system after the decomposition reaction in the first step by volatilization, distillation, or the like.

The reason why such an effect is obtained is believed to be that the decomposing agent represented by Formula (1) is a compound having a low boiling point.

Here, the concept of the "decomposition reaction system after the decomposition reaction in the first step" includes not only the reaction system after the first step and before the second step, but also the reaction system after the first step and during and after the second step.

For example, the decomposition of the polyurea compound in the second step can produce a decomposing agent represented by Formula (1) together with the polyamine compound as a target product. Even in this case, the decomposing agent represented by Formula (1) can be easily removed from the reaction system during and after the second step.

The method of producing a polyamine compound according to the second embodiment further exhibits an excellent ability to remove a decomposing agent represented by Formula (3) from a decomposition reaction system after the decomposition reaction in the second step. For example, the decomposing agent represented by Formula (3) can be easily removed from the reaction system after the decomposition reaction in the second step by volatilization, distillation, or the like.

The reason why such an effect is obtained is believed to be that the decomposing agent represented by Formula (3) is a compound having a low boiling point.

Hereinafter, each step that may be included in the method of producing a polyamine compound according to the second embodiment will be described.

### <First Step>

The first step is a step of generating a polyurea compound by decomposing a thiourethane resin using a decomposing agent represented by Formula (1) above.

For the first step, the generation step in the method of producing a polythiol composition according to the first embodiment can be referred to.

In the first step, a polyurea compound and the polythiol composition can be generated by decomposing a thiourethane resin using a decomposing agent represented by Formula (1) above.

Preferred aspects of the thiourethane resin and the decomposing agent represented by Formula (1) in the first step are the same as preferred aspects of the thiourethane resin and the decomposing agent represented by Formula (1) in the method of producing a polythiol composition according to the first embodiment, respectively.

### (Reaction Pressure)

It is preferable that the first step includes reacting a thiourethane resin with a decomposing agent represented by Formula (1) under a pressure higher than atmospheric pressure. This can further suppress the volatilization of the decomposing agent represented by Formula (1).

In this case, the method includes reacting the thiourethane resin with the decomposing agent represented by Formula (1) under a pressure that is higher than atmospheric pressure by preferably from 0.01 MPa or more (more preferably from 0.01 MPa to 2.0 MPa and still more preferably from 0.02 MPa to 1.0 MPa).

### (Polyurea Compound)

The polyurea compound generated in the first step is an aminolysis decomposition product of a thiourethane resin that is generated by a reaction (i.e., aminolysis) between a thiourethane resin and a decomposing agent represented by Formula (1).

A polyurea compound is a compound that contains two or more urea bonds.

The polyurea compound is, for example, a polyurea compound having a structure in which all isocyanato groups in a polyisocyanate compound, which is one of the raw materials of a thiourethane resin, react with amino groups or monoalkylamino groups in an amine compound A, thereby forming urea bonds. In the disclosure, a polyurea compound having such a structure may be referred to as a urea-form compound of a polyamine compound (e.g., m-xylylenediamine (XDA)) corresponding to a polyisocyanate compound (e.g., m-xylylenediisocyanate (XDI)), which is one of the raw materials, and an amine compound A (e.g., monoethanolamine (MEA)). Here, the polyamine compound corresponding to the polyisocyanate compound means a compound in which all isocyanato groups in the polyisocyanate compound are replaced with amino groups. The polyamine compound corresponding to the polyisocyanate compound is a target product in the method of producing a polyamine compound according to the second embodiment.

### (First Reaction Step and First Separation Step)

The first step may include:
a reaction step of reacting a thiourethane resin and a decomposing agent represented by Formula (1), thereby obtaining a reaction mixture including a polyurea-containing mixture containing a polyurea compound; and
a separation step of separating the polyurea-containing mixture from the reaction mixture.

In this case, in the second step described below, the polyurea-containing mixture separated in the first separation step and a decomposing agent represented by Formula (3) are mixed, thereby reacting the polyurea compound in the polyurea-containing mixture and the decomposing agent represented by Formula (3).

Here, the polyurea-containing mixture means a mixture of two or more urea compounds containing a polyurea compound.

Hereinafter, the reaction mixture, the reaction step, and the separation step described above are also referred to as a first reaction mixture, a first reaction step, and a first separation step, respectively.

Preferred aspects of the reaction in the first reaction step are as described above.

The separation method in the first separation step is not particularly limited, and any known method can be applied.

Examples of the separation method in the first separation step include filtration, decantation, extraction, distillation, drying (including drying under reduced pressure), and purification (e.g., column chromatography). As the separation method, a plurality of methods may be used in combination.

In a case in which the polyurea-containing mixture is generated as a solid content in the first reaction step, the first separation step preferably includes filtrating the first reaction mixture to obtain a polyurea-containing mixture as a residue. The resulting residue may be subjected to washing or other procedures.

In this case, the filtrate obtained by filtering the first reaction mixture may contain a polythiol composition, which is a by-product produced by the aminolysis reaction (more specifically, a by-product when the polyurea-containing mixture is a main product). For preferred aspects of the polythiol composition as a by-product, the above-described preferred aspects of the polythiol composition as a raw material for the thiourethane resin can be referred to, if appropriate.

The polythiol composition produced as a by-product by the aminolysis reaction can be used as a raw material for producing a new thiourethane resin. This allows for the effective use of materials (i.e., recycling).

In a case in which in the first reaction step, a polyurea-containing mixture is produced in a liquid state that is insoluble in a reaction solvent, the first separation step preferably includes separating a supernatant and obtaining a polyurea-containing mixture as an extraction residue by a decantation method that involves repeated extraction and washing. The resulting extraction residue may be subjected to washing or other procedures.

In this case, the supernatant mixture may contain a polythiol composition, which is a by-product produced by the aminolysis reaction (more specifically, a by-product when the polyurea-containing mixture is a main product). For preferred aspects of the polythiol composition as a by-product, the above-described preferred aspects of the polythiol composition as a raw material for the thiourethane resin can be referred to, if appropriate.

The polythiol composition produced as a by-product by the aminolysis reaction can be used as a raw material for producing a new thiourethane resin. This allows for the effective use of materials (i.e., recycling).

In a case in which in the first reaction step, the polyurea-containing mixture is dissolved in the first reaction solvent, the first separation step preferably includes:
filtrating a first reaction mixture to obtain a filtrate;
adding a base containing an alkali metal to the filtrate, and then adding water to perform extraction, thereby removing the alkali metal salt of the polythiol composition as a by-product from the filtrate; and
separating the polyurea-containing mixture from the filtrate from which the alkali metal salt has been removed.

The polyurea-containing mixture can be separated from the filtrate from which the alkali metal salt has been removed by methods such as concentration and drying.

### <Second Step>

The second step is a step of decomposing the polyurea compound produced in the first step with a decomposing agent represented by Formula (3), thereby producing a polyamine compound.

In the second step described below, the polyurea-containing mixture separated in the first separation step and a decomposing agent represented by Formula (3) may be mixed, thereby reacting the polyurea compound in the polyurea-containing mixture and the decomposing agent represented by Formula (3).

The reaction in the second step (second aminolysis) is superior in efficiency of producing a polyamine compound as compared with a known reaction of obtaining a polyamine compound by reacting a polyurea compound with sodium hydroxide. As a result, the amount of the polyamine compound produced can be increased as compared with a case in which the above-described known reactions are applied. Such an effect can be confirmed by analyzing the reaction mixture obtained in the second step by gas chromatography (GC).

### (Reaction Pressure)

It is preferable that the second step includes reacting a polyurea compound with a decomposing agent represented by Formula (3) under a pressure higher than atmospheric pressure. This can further suppress the volatilization of the decomposing agent represented by Formula (3).

In this case, the method includes reacting the polyurea compound with the decomposing agent represented by Formula (3) under a pressure that is higher than atmospheric pressure by preferably from 0.01 MPa or more (more preferably from 0.01 MPa to 2.0 MPa and still more preferably from 0.02 MPa to 1.0 MPa).

### (Decomposing Agent Represented by Formula (3))

The decomposing agent represented by Formula (3) used in the second step is preferably ethylenediamine, N,N'-dimethylethylenediamine, 2-aminoethanol, or ethylene glycol.

In the second step, the charged equivalent of the decomposing agent represented by Formula (3) with respect to the polyurea compound (charged equivalent[decomposing agent represented by Formula (3)/polyurea compound]) is preferably from 0.1 to 50, more preferably from 0.15 to 45, and still more preferably from 0.2 to 40.

In a case in which the charged equivalent[decomposing agent represented by Formula (3)/polyurea compound] is 0.1 or more, the generation of the polyamine compound is further promoted.

In a case in which the charged equivalent[decomposing agent represented by Formula (3)/polyurea compound] is 50 or less, it is advantageous in terms of reducing the amount of the decomposing agent represented by Formula (3) used.

In the second step, in the case of mixing the polyurea-containing mixture separated in the first separation step described above and the decomposing agent represented by Formula (3), thereby reacting the polyurea compound in the polyurea-containing mixture and the decomposing agent represented by Formula (3), the charged equivalent[decomposing agent represented by Formula (3)/polyurea compound] may be determined by regarding the entire amount of the polyurea-containing mixture as the polyurea compound.

In the second step, it is possible to react with the polyurea compound and the decomposing agent represented by Formula (3) in the absence of a reaction solvent. For example, by directly mixing the polyurea-containing mixture containing the polyurea compound and the decomposing agent represented by Formula (3) in the absence of a reaction solvent, it is possible to react with the polyurea compound and the decomposing agent represented by Formula (3) in the absence of a reaction solvent.

Note that in the second step, it is also possible to react with the polyurea compound and the decomposing agent represented by Formula (3) in the presence of a reaction solvent.

### (Second Reaction Temperature)

The temperature of the reaction between the polyurea compound and the decomposing agent represented by Formula (3) in the second step (hereinafter also referred to as "second reaction temperature") is adjusted, if appropriate.

In the second step, it is preferable to react with the polyurea compound and the decomposing agent represented by Formula (3) at a temperature condition (i.e., second reaction temperature) of preferably from 80°C to 200°C (more preferably from 90°C to 200°C, still more preferably from 100°C to 200°C, even still more preferably from 110°C to 190°C, and yet still more preferably from 120°C to 180°C).

In the second step, the reaction may be carried out under pressurized conditions. In a case in which the reaction is carried out under pressurized conditions, the reaction time can sometimes be shortened.

### (Second Reaction Time)

The reaction time of the polyurea compound and the decomposing agent represented by Formula (3) in the second step can be adjusted, if appropriate, but is preferably from 0.1 to 40 hours, more preferably from 0.5 to 20 hours, and still more preferably from 1 to 10 hours.

### (Second Reaction Step and Second Separation Step)

The second step may include:
a reaction step of reacting a polyurea compound and a decomposing agent represented by Formula (3), thereby obtaining a reaction mixture containing the polyamine compound; and
a separation step of separating the polyamine compound from the reaction mixture.

Hereinafter, the reaction mixture, the reaction step, and the separation step described above are also referred to as a second reaction mixture, a second reaction step, and a second separation step, respectively.

Preferred aspects of the reaction in the second reaction step are as described above.

The separation method in the second separation step is not particularly limited, and any known method can be applied.

Examples of the separation method in the second separation step include filtration, decantation, washing, extraction, distillation, pressure reduction (e.g., vacuuming), and purification (e.g., column chromatography). As the separation method, a plurality of methods may be used in combination.

Examples of preferred aspects of the second separation step include an aspect (also referred to as "second separation aspect C") including:
extracting the polyamine compound from the second reaction mixture with a second extraction solvent to obtain an extract; and
separating the polyamine compound from the extract.

In the second separation aspect C, the polyamine compound is separated from the extract obtained by extracting the polyamine compound from the second reaction mixture without directly separating the polyamine compound from the second reaction mixture. This further improves the isolation yield of the final polyamine compound. The reason for this is unclear. However, it is believed that disproportionation in the second separation step (specifically, the reaction in which the polyamine compound returns to the polyurea compound) is more suppressed.

Examples of preferred aspects of the second separation step also include an aspect that includes separating the polyamine compound from the second reaction mixture by distillation.

In the case of separating the polyamine compound from the second reaction mixture by distillation, insoluble components may be removed from the second reaction mixture by filtration, and the polyamine compound may be separated from the second reaction mixture from which the insoluble components have been removed by distillation.

In the case of removing insoluble components from the second reaction mixture by filtration, the second reaction mixture may be diluted before filtration, and the diluted second reaction mixture may be filtrated.

Examples of the second extraction solvent in the second separation aspect C include a hydrocarbon compound.

For example, in the case of using a hydrocarbon compound as the second extraction solvent, preferred aspects of the hydrocarbon compound as the second extraction solvent include the above-described preferred aspects of the hydrocarbon compound as the first reaction solvent. Note that in this case, the hydrocarbon compound as the second extraction solvent and the hydrocarbon compound as the first reaction solvent may be the same or different.

The procedure of separating the polyamine compound from the extract in the second separation aspect C preferably includes distillation.

### (Polyamine Compound as Target Product)

The polyamine compound as a target product in the method of producing a polyamine compound according to the second embodiment is a decomposition product of a polyurea compound generated by the reaction of a polyurea compound and a decomposing agent represented by Formula (3).

The polyamine compound as the target product is preferably a polyamine compound corresponding to the polyisocyanate compound as the raw material of the thiourethane resin, which is the starting material in the first step (more specifically, a compound in which the isocyanato group in the polyisocyanate compound is replaced with an amino group).

The polyamine compound as the target product may be any compound containing two or more amino groups.

The polyamine compound as the target product includes:
preferably a diamine compound containing two amino groups;
preferably at least one selected from the group consisting of pentamethylenediamine, hexamethylenediamine, m-xylylenediamine, p-xylylenediamine, isophoronediamine, bis(aminomethyl)cyclohexane, bis(aminocyclohexyl)methane, 2,5-bis(aminomethyl)bicyclo-[2.2.1]-heptane, 2,6-bis(aminomethyl)bicyclo-[2.2.1]-heptane, tolylenediamine, 4,4'-diphenylmethanediamine, and phenylenediamine (hereinafter also referred to as "polyamine component A"); and
more preferably a polyamine component A as a main component.

The polyamine compound as the target product includes still more preferably at least one selected from the group consisting of m-xylylenediamine, 2,5-bis(aminomethyl)bicyclo-[2.2.1]-heptane, isophorone diisocyanate, bis(isocyanatomethyl)cyclohexane, bis(isocyanatocyclohexyl)methane, and 2,6-bis(aminomethyl)bicyclo-[2.2.1]-heptane (hereinafter also referred to as "polyamine component A1"), and
even still more preferably a polyamine component A1 as a main component.

The use of the polyamine compound as the target product is not particularly limited, and it can be used in a variety of applications.

The polyamine compound as the target product can be used, for example, as a raw material for a polyisocyanate compound.

The produced polyisocyanate compound can be used, for example, in producing a thiourethane resin or a urethane resin.

Specific applications of the produced polyisocyanate compound include polyisocyanate compounds for producing optical materials (for example, lenses).

In other words, a specific example of the method of producing a polyamine compound according to the second embodiment is a method of producing a polyamine compound as a raw material for a polyisocyanate compound for producing an optical material. In this specific example, in the case of using milling swarf containing a thiourethane resin generated during the production of an optical material as a starting material, recycling of the materials (the thiourethane resin and the polyisocyanate compound) can be effectively realized.

### <Other Steps>

The method of producing a polyamine compound according to the second embodiment may include other steps in addition to those described above.

Examples of other steps include a classification step (e.g., a sieving step) and a washing step, which may also be included in the method of producing a polythiol composition according to the first embodiment.

### [Method of Producing Polyisocyanate Compound (Second Embodiment)]

The method of producing a polyisocyanate compound according to the second embodiment of the disclosure includes:
a step of producing a polyamine compound by the method of producing a polyamine compound according to the second embodiment described above; and
a step of reacting at least one of the polyamine compound or a hydrochloride of the polyamine compound with carbonyl dichloride, thereby obtaining a polyisocyanate compound.

According to the method of producing a polyisocyanate compound, a thiourethane resin is used as a starting material and a polyisocyanate compound can be obtained as a target product, thereby realizing recycling of materials (the thiourethane resin and the polyisocyanate compound that is the raw material thereof).

The use of the polyisocyanate compound produced by the method of producing a polyisocyanate compound according to the second embodiment is not particularly limited, and the compound can be used for various purposes.

The polyisocyanate compound can be used, for example, in producing a thiourethane resin or a urethane resin.

Specific examples of the polyisocyanate compound as the target product include the same compounds as the specific examples of the "isocyanate compound as the raw material for the thiourethane resin" in the section "Method of Producing Polythiol Composition."

Specific applications of the polyisocyanate compound as the target product include polyisocyanate compounds for producing optical materials (for example, lenses).

In other words, a specific example of the method of producing a polyisocyanate compound according to the second embodiment is a method of producing a polyisocyanate compound for producing an optical material. In this specific example, in the case of using milling swarf containing a thiourethane resin generated during the production of an optical material as a starting material, effective use (i.e., recycling) of the materials (the thiourethane resin and the polyisocyanate compound) can be effectively realized.

In the step of obtaining a polyisocyanate compound, at least one of the polyamine compound and a hydrochloride of the polyamine compound is reacted with carbonyl dichloride (hereinafter, also referred to as "phosgene") to convert the amino group in the polyamine compound to an isocyanate group, thereby obtaining a polyisocyanate compound.

This reaction itself is known, and known methods of producing polyisocyanate compounds can be referred to, if appropriate.

### [Method of Producing Polymerizable Composition (Second Embodiment)]

The method of producing a polymerizable composition according to the second embodiment of the disclosure includes:
a step of producing a polyisocyanate compound by the method of producing a polyisocyanate compound according to the second embodiment described above; and
a step of mixing at least the polyisocyanate compound and an active hydrogen compound, thereby obtaining a polymerizable composition containing the polyisocyanate compound and the active hydrogen compound.

The method of producing a polymerizable composition according to the second embodiment may include other steps, if necessary.

In the method of producing a polymerizable composition according to the second embodiment,
a polyisocyanate compound is produced using a thiourethane resin (e.g., a thiourethane resin in milling swarf of a thiourethane resin formed body) as a starting material in the step of producing a polyisocyanate compound, and
a polymerizable composition containing the polyisocyanate compound produced above and an active hydrogen compound is produced in the step of obtaining a polymerizable composition.

The obtained polymerizable composition can be reused for producing a thiourethane resin.

In this manner, effective utilization (i.e., recycling) of materials (i.e., the thiourethane resin and the polyisocyanate compound that is the raw material thereof) is realized in the method of producing a polymerizable composition according to the second embodiment.

### <Step of Producing Polyisocyanate Compound>

For the step of producing a polyisocyanate compound, the method of producing a polyisocyanate compound according to the second embodiment described above can be referred to, if appropriate.

### <Step of Obtaining Polymerizable Composition>

In the step of obtaining a polymerizable composition, the polymerizable composition containing the polyisocyanate compound and the active hydrogen compound described above is obtained by mixing at least the polyisocyanate compound and the active hydrogen compound.

Examples of the active hydrogen compound include a polythiol compound, a polyol compound, and a polyamine compound.

The active hydrogen compound may be of only one kind or of two or more kinds.

The active hydrogen compound is preferably a polythiol composition.

Preferred aspects of the polythiol composition as an active hydrogen compound are the same as the preferred aspects of the "polythiol composition as a raw material for a thiourethane resin" explained in the section "Method of Producing Polyamine Compound."

In the step of obtaining a polymerizable composition, the mixing ratio of the active hydrogen compound and the polyisocyanate compound is not particularly limited.

In the step of obtaining a polymerizable composition, the ratio of the charged mass of the active hydrogen compound to the charged mass of the polyisocyanate compound (i.e., charged mass[active hydrogen compound/polyisocyanate compound]) is preferably from 0.10 to 10.0, more preferably from 0.20 to 5.00, still more preferably from 0.50 to 1.50, and even sill more preferably from 0.70 to 1.30.

It is further preferable that the molar ratio of the mercapto group of the polythiol compound to the isocyanato group of the polyisocyanate compound contained in the polythiol composition (mercapto group/isocyanato group) is preferably from 0.5 to 3.0, more preferably from 0.6 to 2.0, and more preferably from 0.8 to 1.3.

In the step of obtaining a polymerizable composition, the total charged mass of the active hydrogen compound and the polyisocyanate compound is not particularly limited. However, it is preferably 60% by mass or more, more preferably 80% by mass or more, and still more preferably 90% by mass or more with respect to the total amount of the polymerizable composition to be produced.

In the step of obtaining a polymerizable composition, at least the polyisocyanate compound and the active hydrogen compound described above are mixed. However, if appropriate, the polyisocyanate compound, the active hydrogen compound, and other components may be mixed.

In addition, in the step of obtaining a polymerizable composition, at least the polyisocyanate compound and the active hydrogen compound may be mixed, and then other components may be mixed into the mixture.

For other components, the other components used in the method of producing a polymerizable composition according to the first embodiment can be referred to.

In the step of obtaining a polymerizable composition, the above-described components can be mixed by an ordinary method, and the mixing method is not particularly limited.

### [Method of Producing Resin (Second Embodiment)]

The method of producing a resin according to the second embodiment of the disclosure includes:
a step of producing a polymerizable composition by the above-described method of producing a polymerizable composition according to the second embodiment; and
a step of obtaining a resin by curing the polymerizable composition.

The method of producing a resin according to the second embodiment and preferred aspects thereof are the same as the method of producing a resin according to the first embodiment and preferred aspects thereof, except that a polymerizable composition is produced by the method of producing a polymerizable composition according to the second embodiment, instead of producing a polymerizable composition by the method of producing a polymerizable composition according to the first embodiment.

### [Method of Producing Formed body (Second Embodiment)]

The method of producing a formed body according to the second embodiment of the disclosure is a method of producing a formed body containing a resin, the method including:
a step of producing a polymerizable composition by the above-described method of producing a polymerizable composition according to the second embodiment; and
a step of obtaining a formed body containing a resin by curing the polymerizable composition.

The method of producing a formed body according to the second embodiment and preferred aspects thereof are the same as the method of producing a formed body according to the first embodiment and preferred aspects thereof, except that a polymerizable composition is produced by the method of producing a polymerizable composition according to the second embodiment, instead of producing a polymerizable composition by the method of producing a polymerizable composition according to the first embodiment.

### [Method of Producing Optical Material (Second Embodiment), Method of Producing Lens (Second Embodiment)]

The method of producing an optical material (e.g., lens) according to the second embodiment of the disclosure is a method of producing an optical material (e.g., lens) including a formed body containing a resin, the method including:
a step of producing a polymerizable composition by the above-described method of producing a polymerizable composition according to the second embodiment; and
a step of obtaining a formed body containing a resin by curing the polymerizable composition.

The method of producing an optical material (e.g., lens; the same applies below) according to the second embodiment may include other steps as necessary.

The method of producing an optical material (e.g., lens) according to the second embodiment and preferred aspects thereof are the same as the method of producing an optical material (e.g., lens) according to the first embodiment and preferred aspects thereof, except that a polymerizable composition is produced by the method of producing a polymerizable composition according to the second embodiment, instead of producing a polymerizable composition by the method of producing a polymerizable composition according to the first embodiment.

### [Resin (Second Embodiment), Formed body (Second Embodiment), Optical Material (e.g., Lens) (Second Embodiment)]

The resin according to the second embodiment of the disclosure is a cured product of the polymerizable composition according to the second embodiment of the disclosure described above.

The formed body according to the second embodiment of the disclosure is a formed body containing the resin according to the second embodiment described above.

The optical material (e.g., lens) according to the second embodiment is an optical material (e.g., lens) containing the resin according to the second embodiment described above.

The resin, formed body, and optical material (e.g., lens) according to the second embodiment and preferred aspects thereof are the same as the resin, formed body, and optical material (e.g., lens) according to the first embodiment and preferred aspects thereof, respectively, except that a polymerizable composition is produced by the method of producing a polymerizable composition according to the second embodiment, instead of producing a polymerizable composition by the method of producing a polymerizable composition according to the first embodiment.

### <<Third Embodiment>>

### [Method of Producing Polyamine Compound (Third Embodiment)]

The method of producing a polyamine compound according to the third embodiment includes:
Step X1 of generating a polycarbamate compound by decomposing a thiourethane resin using a decomposing agent represented by the following Formula (2) in the presence of a tertiary amine compound serving as a decomposition aid; and
Step X2 of generating a polyamine compound by decomposing the polycarbamate compound using a decomposing agent represented by the following Formula (3):

   R³-OH (2)

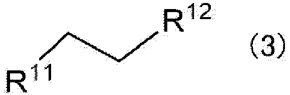

In Formula (2), R³ represents an alkyl group having from 1 to 3 carbon atoms.

In Formula (3), R¹¹ and R¹² each independently represent a hydroxy group, a mercapto group, an amino group, or a monomethylamino group.

The method of producing a polyamine compound according to the third embodiment exhibits an excellent ability to remove a decomposing agent represented by Formula (2) from a decomposition reaction system after the decomposition reaction in Step X1. For example, the decomposing agent represented by Formula (2) can be easily removed from the reaction system after the decomposition reaction in Step X1 by volatilization, distillation, or the like.

The reason why such an effect is obtained is believed to be that the decomposing agent is a compound having a low boiling point.

Here, the concept of the "decomposition reaction system after the decomposition reaction in Step X1" includes not only the reaction system after Step X1 and before Step X2, but also the reaction system after Step X1 and during and after Step X2.

For example, the decomposition of the polycarbamate compound in Step X2 can produce a decomposing agent represented by Formula (2) together with the polyamine compound as a target product. Even in this case, the decomposing agent represented by Formula (2) can be easily removed from the reaction system during and after Step X2.

The method of producing a polyamine compound according to the third embodiment further exhibits an excellent ability to remove also a decomposing agent represented by Formula (3) from a decomposition reaction system after the decomposition reaction in Step X2. For example, the decomposing agent represented by Formula (3) can be easily removed from the reaction system after the decomposition reaction in Step X2 by volatilization, distillation, or the like.

The reason why such an effect is obtained is believed to be that the decomposing agent represented by Formula (3) is a compound having a low boiling point.

Hereinafter, each step that may be included in the method of producing a polyamine compound according to the third embodiment will be described.

### <Step X1>

Step X1 is a step of generating a polycarbamate compound by decomposing a thiourethane resin using a decomposing agent represented by the following Formula (2) in the presence of a tertiary amine compound serving as a decomposition aid.

For Step X1, the generation step in the method of producing a polythiol composition according to the first embodiment can be referred to.

In Step X1, a thiourethane resin is decomposed by a decomposing agent represented by the following Formula (2) in the presence of a tertiary amine compound as a decomposition aid, thereby generating a polycarbamate compound and the above-described polythiol composition.

Preferred aspects of the thiourethane resin, the decomposing agent represented by Formula (2), and the tertiary amine compound as a decomposition aid in Step X1 are the same as preferred aspects of the thiourethane resin, the decomposing agent represented by Formula (2), and the tertiary amine compound as a decomposition aid in the method of producing a polythiol composition according to the first embodiment, respectively.

### (Reaction Pressure)

It is preferable that Step X1 includes reacting a thiourethane resin and a decomposing agent represented by Formula (2) under a pressure higher than atmospheric pressure in the presence of tertiary amine compound as a decomposition aid. This can further suppress the volatilization of the decomposing agent represented by Formula (2).

In this case, the method includes reacting the thiourethane resin with the decomposing agent represented by Formula (2) under a pressure that is higher than atmospheric pressure by preferably from 0.01 MPa or more (more preferably from 0.01 MPa to 2.0 MPa and still more preferably from 0.02 MPa to 1.0 MPa).

### (Polycarbamate Compound)

The polycarbamate compound generated in Step X1 is an alcohololysis decomposition product of a thiourethane resin that is generated by a reaction (i.e., alcohololysis) between a thiourethane resin and a decomposing agent represented by Formula (2).

A polycarbamate compound is a compound that contains two or more carbamate bonds (i.e., urethane bonds) (i.e., polyurethane compound).

The polycarbamate compound is, for example, a polycarbamate compound (i.e., a polyurethane compound) having a structure in which all isocyanato groups in a polyisocyanate compound, which is one of the raw materials of a thiourethane resin, react with hydroxy groups in an alcohol compound, thereby forming carbamate bonds (i.e., urethane bonds). In the disclosure, a polycarbamate compound having such a structure may be referred to as a carbamate-form compound of a polyamine compound (e.g., XDA) corresponding to a polyisocyanate compound (e.g., XDI), which is one of the raw materials, and an alcohol compound (e.g., 1-octanol). Here, the polyamine compound corresponding to the polyisocyanate compound means a compound in which all isocyanato groups in the polyisocyanate compound are replaced with amino groups. The polyamine compound corresponding to the polyisocyanate compound is a target product in the method of producing a polyamine compound according to the third embodiment.

### (Reaction Step X1 and Separation Step X2)

Step X1 may include:
a reaction step of reacting a thiourethane resin and a decomposing agent represented by Formula (2), thereby obtaining a reaction mixture including a polycarbamate-containing mixture containing a polycarbamate compound; and
a separation step of separating the polycarbamate-containing mixture from the reaction mixture.

In this case, in Step X2 described below, the polycarbamate-containing mixture separated in the separation step and a decomposing agent represented by Formula (3) are mixed, thereby reacting the polycarbamate compound in the polycarbamate-containing mixture and the decomposing agent represented by Formula (3).

Here, the polycarbamate-containing mixture means a mixture of two or more carbamate compounds containing a polycarbamate compound.

Hereinafter, the reaction mixture, the reaction step, and the separation step described above are also referred to as a reaction mixture X1, Reaction Step X1, and Separation Step X2, respectively.

The preferred conditions for Reaction Step X1 are as described above.

The reaction mixture produced in Reaction Step X1 may contain a polycarbamate-containing mixture as a main product produced by alcoholysis, and other components other than the polycarbamate-containing mixture.

Examples of other components include a by-product produced by alcoholysis (e.g., polythiol composition), the reaction solvent described above, residues of the raw materials (thiourethane resin and/or alcohol compound), and impurities contained in the raw materials.

For preferred aspects of the polythiol composition as a by-product, the above-described preferred aspects of the polythiol composition as a raw material for the thiourethane resin can be referred to, if appropriate.

The polythiol composition produced as a by-product by the alcohololysis reaction can be used as a raw material for producing a new thiourethane resin. This allows for the recycling of materials.

The separation method in Separation Step X2 is not particularly limited, and any known method can be applied.

Examples of the separation method in Separation Step X2 include filtration, decantation, extraction, distillation, drying (including drying under reduced pressure), and purification (e.g., column chromatography). As the separation method, a plurality of methods may be used in combination.

For example, one preferred aspect of Separation Step X2 includes:
filtrating a reaction mixture X1 to obtain a filtrate;
washing the filtrate with an acid and then with water;
adding a base containing an alkali metal to the filtrate washed with water, and then washing the filtrate with water to remove a salt (e.g., alkali metal salt of the polythiol composition, which is a reaction by-product); and
separating the polycarbamate-containing mixture from the filtrate from which the salt has been removed (hereinafter referred to as "separation aspect X1").

In the separation aspect X1, first, the filtrate is washed with an acid (hereinafter also referred to as acid washing), thereby removing an amine (e.g., tertiary amine compound) from the filtrate. Examples of the acid used for the acid washing include hydrochloric acid, carbonic acid, nitric acid, sulfuric acid, acetic acid, formic acid, and oxalic acid.

The acid-washed filtrate is then washed with water, thereby removing the acid from the filtrate. At a stage where the acid has been removed, the filtrate contains the polycarbamate-containing mixture as a target product. However, it is believed that the filtrate may also contain a polythiol composition as a by-product.

In separation aspect X1, a base containing an alkali metal is added to the filtrate washed with water. This converts the polythiol composition in the filtrate into an alkali metal salt. The filtrate is then washed with water, thereby removing the alkali metal salt of the polythiol composition from the filtrate.

In the separation aspect X1, the polycarbamate-containing mixture is separated by a known method from the filtrate from which the alkali metal salt of the polythiol composition has been removed.

In separation aspect X1, the alkali metal in the base containing an alkali metal is preferably sodium, potassium, or lithium, and more preferably sodium or potassium.

Examples of the base containing an alkali metal include sodium methoxide, sodium ethoxide, sodium propoxide, sodium hydroxide, potassium hydroxide, and lithium hydroxide.

The base containing an alkali metal can be added to the filtrate in the form of an alcohol solution (methanol solution, ethanol solution, or the like) if necessary.

In addition, in separation aspect X1, before the filtrate obtained by filtrating the reaction mixture X1 is washed with an acid, a solvent (hereinafter also referred to as separation solvent X1) may be added to the filtrate, and the filtrate to which the separation solvent X1 has been added may be sequentially subjected to acid washing and water washing.

As the separation solvent X1, a solvent similar to the reaction solvent may be used, or an alcohol solvent may be used.

In a case in which a reaction solvent is used in Reaction Step X1, the addition of the separation solvent X1 to the filtrate may be omitted.

### <Step X2>

Step X2 is a step of decomposing the polycarbamate compound produced in Step X1 with a decomposing agent represented by Formula (3), thereby producing a polyamine compound.

In Step X2 described below, the polycarbamate-containing mixture separated in the Separation Step X2 and a decomposing agent represented by Formula (3) may be mixed, thereby reacting the polycarbamate compound in the polycarbamate-containing mixture and the decomposing agent represented by Formula (3).

The reaction in Step X2 is superior in efficiency of producing a polyamine compound as compared with a known reaction of obtaining a polyamine compound by reacting a polycarbamate compound with sodium hydroxide. As a result, the amount of the polyamine compound produced can be increased as compared with a case in which the above-described known reactions are applied. Such an effect can be confirmed by analyzing the reaction mixture obtained in Step X2 by gas chromatography (GC).

Step X2 in the method of producing a polyamine compound according to the third embodiment and preferred aspects thereof are the same as the second step in the method of producing a polyamine compound according to the third embodiment and preferred aspects thereof, except that an object to be decomposed by a decomposing agent represented by Formula (3) is a polycarbamate compound generated in Step X1 in the third embodiment, but not a polyurea compound generated in the first step in the second embodiment.

### (Reaction Pressure)

It is preferable that Step X2 includes reacting a polycarbamate compound with a decomposing agent represented by Formula (3) under a pressure higher than atmospheric pressure. This can further suppress the volatilization of the decomposing agent represented by Formula (3).

In this case, the method includes reacting the polycarbamate compound with the decomposing agent represented by Formula (3) under a pressure that is higher than atmospheric pressure by preferably from 0.01 MPa or more (more preferably from 0.01 MPa to 2.0 MPa and still more preferably from 0.02 MPa to 1.0 MPa).

### (Polyamine Compound as Target Product)

The polyamine compound as a target product in the method of producing a polyamine compound according to the third embodiment is a decomposition product of a thiourethane resin generated by the reaction of a polycarbamate compound and a decomposing agent represented by Formula (3).

The polyamine compound as the target product is preferably a polyamine compound corresponding to the polyisocyanate compound as the raw material of the thiourethane resin, which is the starting material in Step X1 (more specifically, a compound in which the isocyanato group in the polyisocyanate compound is replaced with an amino group).

Preferred aspects and use applications of the polyamine compound as the target product in the third embodiment are the same as preferred aspects and use applications of the polyamine compound as the target product in the third embodiment.

### <Other Steps>

The method of producing a polyamine compound according to the third embodiment may include other steps in addition to those described above.

Examples of other steps include a classification step (e.g., a sieving step) and a washing step, which may also be included in the method of producing a polythiol composition according to the first embodiment.

### [Method of Producing Polyisocyanate Compound (Third Embodiment)]

The method of producing a polyisocyanate compound according to the third embodiment of the disclosure includes:
a step of producing a polyamine compound by the method of producing a polyamine compound according to the third embodiment described above; and
a step of reacting at least one of the polyamine compound or a hydrochloride of the polyamine compound with carbonyl dichloride, thereby obtaining a polyisocyanate compound.

The method of producing a polyisocyanate compound according to the third embodiment and preferred aspects thereof are the same as the method of producing a polyisocyanate compound according to the second embodiment and preferred aspects thereof, except that a polyamine compound is produced by the method of producing a polyamine compound according to the third embodiment, instead of producing a polyamine compound by the method of producing a polyamine compound according to the second embodiment.

### [Method of Producing Polymerizable Composition (Second Embodiment)]

The method of producing a polymerizable composition according to the third embodiment of the disclosure includes:
a step of producing a polyisocyanate compound by the method of producing a polyisocyanate compound according to the third embodiment described above; and
a step of mixing at least the polyisocyanate compound and an active hydrogen compound, thereby obtaining a polymerizable composition containing the polyisocyanate compound and the active hydrogen compound.

The method of producing a polymerizable composition according to the third embodiment and preferred aspects thereof are the same as the method of producing a polymerizable composition according to the second embodiment and preferred aspects thereof, except that a polyisocyanate compound is produced by the method of producing a polyisocyanate compound according to the third embodiment, instead of producing a polyisocyanate compound by the method of producing a polyisocyanate compound according to the second embodiment.

### [Method of Producing Resin (Third Embodiment)]

The method of producing a resin according to the third embodiment of the disclosure includes:
a step of producing a polymerizable composition by the above-described method of producing a polymerizable composition according to the third embodiment; and
a step of obtaining a resin by curing the polymerizable composition.

The method of producing a resin according to the third embodiment and preferred aspects thereof are the same as the method of producing a resin according to the second embodiment and preferred aspects thereof, except that a polymerizable composition is produced by the method of producing a polymerizable composition according to the third embodiment, instead of producing a polymerizable composition by the method of producing a polymerizable composition according to the second embodiment.

### [Method of Producing Formed body (Second Embodiment)]

The method of producing a formed body according to the second embodiment of the disclosure is a method of producing a formed body containing a resin, the method including:
a step of producing a polymerizable composition by the above-described method of producing a polymerizable composition according to the second embodiment; and
a step of obtaining a formed body containing a resin by curing the polymerizable composition.

The method of producing a formed body according to the second embodiment and preferred aspects thereof are the same as the method of producing a formed body according to the first embodiment and preferred aspects thereof, except that a polymerizable composition is produced by the method of producing a polymerizable composition according to the second embodiment, instead of producing a polymerizable composition by the method of producing a polymerizable composition according to the first embodiment.

### [Method of Producing Optical Material (Third Embodiment), Method of Producing Lens (Third Embodiment)]

The method of producing an optical material (e.g., lens) according to the third embodiment of the disclosure is a method of producing an optical material (e.g., lens) including a formed body containing a resin, the method including:
a step of producing a polymerizable composition by the above-described method of producing a polymerizable composition according to the third embodiment; and
a step of obtaining a formed body containing a resin by curing the polymerizable composition.

The method of producing an optical material (e.g., lens) according to the third embodiment and preferred aspects thereof are the same as the method of producing an optical material (e.g., lens) according to the second embodiment and preferred aspects thereof, except that a polymerizable composition is produced by the method of producing a polymerizable composition according to the third embodiment, instead of producing a polymerizable composition by the method of producing a polymerizable composition according to the second embodiment.

### [Resin (Third Embodiment), Formed body (Third Embodiment), Optical Material (e.g., Lens) (Third Embodiment)]

The resin according to the third embodiment of the disclosure is a cured product of the polymerizable composition according to the third embodiment of the disclosure described above.

The formed body according to the third embodiment of the disclosure is a formed body containing the resin according to the third described above.

The optical material (e.g., lens) according to the third embodiment is an optical material (e.g., lens) containing the resin according to the third embodiment described above.

The resin, formed body, and optical material (e.g., lens) according to the third embodiment and preferred aspects thereof are the same as the resin, formed body, and optical material (e.g., lens) according to the second embodiment and preferred aspects thereof, respectively, except that a polymerizable composition is produced by the method of producing a polymerizable composition according to the third embodiment, instead of producing a polymerizable composition by the method of producing a polymerizable composition according to the second embodiment.

### EXAMPLES

Examples of the disclosure will be described below, but the disclosure is not limited to the following Examples.

Hereinafter, unless otherwise specified, "part(s)" are based on mass, and "room temperature" is 25°C.

### <Resin Performance Test>

As a performance test of a resin, a performance test was carried out on a flat formed body having a thickness of 2.5 mm.

The performance test items are as follows.

### • Yellow Index (YI)

The yellow index was measured using a SPECTROPHOTOMETER CM-5 manufactured by Konica Minolta, Inc.

### • L*, a*, and b*

Using a SPECTROPHOTOMETER CM-5 manufactured by Konica Minolta, Inc., L*, a*, and b* in the CIE1976 (L*, a*, b*) color system were measured.

### • Refractive index (ne) and Abbe number (ve)

Using a PULFRICH REFRACTOMETER KPR-30 manufactured by SHIMADZU CORPORATION, the refractive indices (ne, nF', nC') were measured at 20°C at wavelengths of 546.1 nm (mercury e-line), 480.0 nm (Cd F' line), and 643.9 nm (Cd C' line). Based on these measurement results, the refractive index (ne) and the Abbe number (ve) were determined.

### • Heat resistance

The glass transition temperature (Tg) was measured by the TMA penetration method (load: 50 g; pin tip: 0.5 mmφ; heating rate: 10°C/min) using a THERMOMECHANICAL ANALYZER TMA-60 manufactured by SHIMADZU CORPORATION, and was used as an index of heat resistance.

### • Specific gravity d

Specific gravity d was measured by the Archimedes method at 20°C.

### [Reference Production Example 1]

### <Production of Formed body Containing Thiourethane Resin R1>

A flask equipped with a stirrer was charged with:
dibutyltin dichloride as a polymerization catalyst (100 ppm by mass based on the total amount of the polyisocyanate compound and the polythiol composition);
ZELEC UN (manufactured by Stepan Corporation; acidic phosphate ester) as a mold release agent (1000 ppm by mass based on the total amount of the polyisocyanate compound and the polythiol composition);
m-xylylene diisocyanate (XDI) as a polyisocyanate compound (52 parts by mass); and
a polythiol composition (A) (48 parts by mass) containing 4-mercaptomethyl-1,8-dimercapto-3,6-dithiaoctane (i.e., polythiol component A1) as a main component,
followed by stirring at room temperature (25°C) for 1 hour. Thus, a polymerizable composition was obtained as a transparent homogeneous solution. Here, the polythiol composition (A) used was one that was produced in Reference Production Example 3 described below.

Next, the polymerizable composition was filtrated under reduced pressure using a polytetrafluoroethylene (PTFE) filter, and then thoroughly degassed under a reduced pressure of 600 Pa until no foaming was observed. The degassed polymerizable composition was poured between a pair of glass molds fixed with tape, and then the pair of glass molds was placed in an oven, and the temperature inside the oven was set to 10°C. Next, the temperature inside the oven was raised from 10°C to 120°C over 38 hours. Through the above process, the monomers (polyisocyanate compound and polythiol composition) in the degassed polymerizable composition were polymerized, and a formed body containing thiourethane resin R1 (i.e., a cured product of the polymerizable composition) was formed between the pair of glass molds (thickness: 9.0 mm).
Subsequently, the inside of the oven was cooled, and after cooling, the pair of glass molds was taken out of the oven, and then the formed body was removed from the pair of glass molds, thereby obtaining a formed body.

### [Reference Production Example 2]

### <Production of Thiourethane Resin Swarf R1>

The formed body obtained in Reference Production Example 1 was cut, thereby producing a lens. The milling swarf generated during this process was collected, thereby obtaining thiourethane resin swarf R1 (i.e., resin swarf containing thiourethane resin R1).

### [Reference Production Example 3]

### <Production of Polythiol Composition (A)>

A reactor was charged with 125.4 parts by mass of 2-mercaptoethanol and 18.3 parts by mass of degassed water. To the reactor, 99.8 parts by mass of 32% by mass aqueous solution of sodium hydroxide was added dropwise over 40 minutes from 12°C to 35°C, and then 73.8 parts by mass of epichlorohydrin was added dropwise from 29°C to 36°C over 4 hours, followed by stirring for 30 minutes. From the NMR data, the generation of 1,3-bis(2-hydroxyethylthio)-2-propanol was confirmed.

The reactor, in which the generation of 1,3-bis(2-hydroxyethylthio)-2-propanol was confirmed, was charged with 332.0 parts by mass of 36% by mass hydrochloric acid, and then with 183.8 parts by mass of thiourea having a purity of 99.9%. The mixture was stirred under reflux at 110°C for 3 hours, thereby carrying out a thiuronium salt reaction. After the thiuronium chloride reaction, the inside of the reactor was cooled to 45°C, and then 355.0 parts by mass of toluene was added thereto. The mixture was then cooled to 30°C, and then 244.6 parts by mass of a 25% by mass aqueous ammonia solution was charged from 30°C to 40°C over 44 minutes. The hydrolysis reaction was then carried out with stirring from 54°C to 62°C for 3 hours. This hydrolysis reaction yielded a toluene solution of a polythiol composition (A) containing 4-mercaptomethyl-1,8-dimercapto-3,6-dithiaoctane (i.e., polythiol component A1) as a main component.

To the obtained toluene solution, 147.8 parts by mass of 36% by mass hydrochloric acid was added, and the mixture was acid-washed from 35°C to 40°C for 1 hour. After the acid washing, the aqueous phase was extracted, and then 147.8 parts by mass of degassed water was added to the remaining organic phase, followed by washing once from 35°C to 40°C for 10 minutes. To the organic phase after washing with degassed water, 147.8 parts by mass of 0.1% by mass ammonia water was added, and washing was carried out for 10 minutes. To the organic phase after washing with ammonia water, 147.8 parts by mass of degassed water was added, and washing was carried out twice from 35°C to 40°C for 10 minutes. From the organic phase after being washed twice, toluene and a trace amount of water were removed under reduced pressure while heating, and then the mixture was filtrated under reduced pressure using a 3.0 µm PTFE-type membrane filter, thereby obtaining 200.0 parts by mass of a polythiol composition (A) mainly composed of 4-mercaptomethyl-1,8-dimercapto-3,6-dithiaoctane (i.e., polythiol component A1).

The purity of the polythiol component A1 in the polythiol composition (A) obtained in Reference Production Example 3 was 91.6%.

### [Example 1]

### <Decomposition of Thiourethane Resin (Aminolysis with Ammonia as Decomposing Agent Represented by Formula (1))>

To a 100-mL PRESSURE-RESISTANT REACTION VESSEL manufactured by SAN-AI Kagaku Co. Ltd.,
thiourethane resin swarf R1 (18.0 g) obtained in Reference Production Example 2, toluene (31.9 g), and
25% ammonia water (10.1 g) (corresponding to 0.15 mol of ammonia as the decomposing agent represented by Formula (1)) were added, thereby obtaining a reaction solution. Nitrogen gas was then filled into the reaction vessel, and the vessel was sealed.

Next, the reaction solution in the reaction vessel was heated to 90°C under a sealed condition, and stirred for 6 hours under a pressure condition at least 0.01 MPa higher than atmospheric pressure, thereby carrying out a decomposition reaction of the thiourethane resin.

After the decomposition reaction was completed, the reaction solution was cooled to room temperature and filtrated under reduced pressure.

By the above-described filtration under reduced pressure, 10.1 g (yield: 91.4%) of a polyurea compound B1 was obtained as a residue, and a toluene solution containing a polythiol composition was obtained as a filtrate.

Here, the polyurea compound B1 and the polythiol composition are both decomposition products produced by aminolysis of the thiourethane resin swarf R1.

During the above-described process of filtration under reduced pressure, ammonia as the decomposing agent represented by Formula (1) was volatilized and easily removed from the reaction system.

To the filtrate (i.e., the toluene solution containing the polythiol composition), 30.0 g of a 35% aqueous hydrochloric acid solution was added, followed by acid washing. The toluene solution after the acid washing was washed twice with water by adding 30.0 g of degassed water, and then 12.4 g of a 32% aqueous sodium hydroxide solution and 20 g of degassed water were added sequentially, thereby obtaining an aqueous solution containing an alkali metal salt of polythiol as the aqueous phase.

The aqueous solution containing the alkali metal salt of polythiol was washed twice with 20.0 g of toluene. Next, 40.0 g of toluene was added to the washed aqueous solution, and then 30.0 g of a 35% aqueous hydrochloric acid solution was added thereto for neutralization. The aqueous phase was extracted from the neutralized liquid, and 30.0 g of degassed water was added to the remaining organic phase, followed by liquid separation and washing performed twice, thereby obtaining a toluene solution in which the polythiol composition was dissolved.

From the obtained toluene solution, toluene and a trace amount of water were removed under reduced pressure while heating, and then the mixture was filtrated under reduced pressure using a 3.0 µm PTFE-type membrane filter, thereby obtaining 6.9 g (yield: 79.9%) of a polythiol composition (A) mainly composed of 4-mercaptomethyl-1,8-dimercapto-3,6-dithiaoctane (i.e., polythiol component A1).

### [Example 2]

### <Decomposition of Thiourethane Resin (Aminolysis with Methylamine as Decomposing Agent Represented by Formula (1))>

The same procedures as in Example 1 were carried out, except that "25% ammonia water (10.1 g) (corresponding to 0.15 mol of ammonia as a decomposing agent represented by Formula (1))" in Example 1 was changed to a "40% methylamine aqueous solution (1.6 g) (corresponding to 0.15 mol of methylamine as a decomposing agent represented by Formula (1))."

In Example 2, by the filtration under reduced pressure after the completion of the decomposition reaction, 10.9 g (yield: 87.6%) of a polyurea compound B2 was obtained as a residue, and a toluene solution containing a polythiol composition was obtained as a filtrate.

Here, the polyurea compound B2 and the polythiol composition are both decomposition products produced by aminolysis of the thiourethane resin swarf R1.

During the above-described process of filtration under reduced pressure, methylamine as the decomposing agent represented by Formula (1) was volatilized and easily removed from the reaction system.

The toluene solution containing the polythiol composition was treated in the same manner as in Example 1, thereby obtaining 7.01 g (yield: 81.1%) of a polythiol composition (A) mainly composed of 4-mercaptomethyl-1,8-dimercapto-3,6-dithiaoctane (i.e., polythiol component A1).

### [Example 3]

### <Decomposition of Thiourethane Resin (Aminolysis with Dimethylamine as Decomposing Agent Represented by Formula (1))>

The same procedures as in Example 1 were carried out, except that "25% ammonia water (10.1 g) (corresponding to 0.15 mol of ammonia as a decomposing agent represented by Formula (1))" in Example 1 was changed to a "50% dimethylamine aqueous solution (13.4 g) (corresponding to 0.15 mol of dimethylamine as a decomposing agent represented by Formula (1))."

In Example 3, after the completion of the decomposition reaction, liquid separation and extraction were performed, thereby obtaining an aqueous solution of a polyurea compound B3, and then water was distilled off from the obtained aqueous solution, thereby obtaining 12.8 g of the polyurea compound B3 (yield: 95.0%). By the liquid separation and extraction, a toluene solution containing a polythiol composition was obtained as an organic phase.

Here, the polyurea compound B2 and the polythiol composition are both decomposition products produced by aminolysis of the thiourethane resin swarf R1.

In the above-described liquid separation and extraction process, dimethylamine as the decomposing agent represented by Formula (1) was removed from the reaction system.

### [Example 4]

### <Decomposition of Thiourethane Resin (Alcohololysis with Methanol as Decomposing Agent Represented by Formula (2))>

To a 100-mL PRESSURE-RESISTANT REACTION VESSEL manufactured by SAN-AI Kagaku Co. Ltd.,
thiourethane resin R1 (18.0 g) obtained in Reference Production Example 2,
31.8 g (1.0 mol) of methanol as a decomposing agent represented by Formula (2), and
1,4-diazabicyclo-[2,2,2]-octane (1.1 g; 0.010 mol) as a tertiary amine (decomposition aid) were added, thereby obtaining a reaction solution.
Nitrogen gas was then filled into the reaction vessel, and the vessel was sealed.

Next, the reaction solution in the reaction vessel was heated to 150°C under a sealed condition, and stirred for 9 hours under a pressure condition at least 0.01 MPa higher than atmospheric pressure, thereby carrying out a decomposition reaction of the thiourethane resin.

After the completion of the decomposition reaction, insoluble matter was removed from the reaction solution by filtration under reduced pressure, and 30.0 g of 2-octanol was added thereto, followed by the addition of 30.0 g of a 35% aqueous hydrochloric acid solution for acid washing. The reaction solution after the acid washing was washed twice repeatedly with water by adding 30.0 g of degassed water, and then 12.4 g of a 32% aqueous sodium hydroxide solution and 20 g of degassed water were added sequentially, thereby obtaining an organic phase and an aqueous phase.

The alcohol solution in which the polycarbamate compound was dissolved was recovered as the organic phase, and the solvent was distilled off from the recovered organic phase under heating and reduced pressure, thereby obtaining 7.10 g (yield: 56.6%) of a polycarbamate compound C1.

### Meanwhile, an aqueous solution containing an alkali metal salt of polythiol was obtained as an aqueous phase.

The aqueous solution containing the alkali metal salt of polythiol was washed twice with 20.0 g of toluene. Next, 40.0 g of toluene was added to the washed aqueous solution, and then 30.0 g of a 35% aqueous hydrochloric acid solution was added thereto for neutralization. The aqueous phase was extracted from the neutralized liquid, and 30.0 g of degassed water was added to the remaining organic phase, followed by liquid separation and washing performed twice, thereby obtaining a toluene solution in which the polythiol composition was dissolved.

From the obtained toluene solution, toluene and a trace amount of water were removed under reduced pressure while heating, and then the mixture was filtrated under reduced pressure using a 3.0 µm PTFE-type membrane filter, thereby obtaining 5.1 g (yield: 59.0%) of a polythiol composition (A) mainly composed of 4-mercaptomethyl-1,8-dimercapto-3,6-dithiaoctane (i.e., polythiol component A1).

In Example 4, during the above-described process of filtration under reduced pressure after the completion of the decomposition reaction, methanol as the decomposing agent represented by Formula (2) was volatilized and easily removed from the reaction system.

### <Measurement of Polythiol Compositions (A)>

The polythiol compositions (A) obtained in Examples 1 to 4 were subjected to the following measurements.

The results are shown in Table 1.

### (Purity (%) of Polythiol Component A1 in Polythiol Composition (A))

The purity (%) of the polythiol component A1 in each polythiol composition (A) was measured by the above-described method using high-performance liquid chromatography.

### (Refractive Index)

The refractive index of the polythiol composition (A) was measured using a liquid refractometer RA600 manufactured by Kyoto Electronics Manufacturing Co., Ltd.

### (Thiol Value)

The thiol value of the polythiol composition (A) was determined by oxidation-reduction titration using a 0.05 M aqueous iodine solution.

**[Table 1]**

| | | Example 1 | Example 2 | Example 3 | Example 4 |
|---|---|---|---|---|---|
| Polythiol composition (A) | Purity of polythiol component A1 (%) | 89.92 | 84.23 | 78.92 | 84.15 |
| | Refractive index | 1.6314 | 1.6302 | 1.6297 | 1.6304 |
| | Thiol value [mmol/g] | 11.24 | 11.01 | 10.77 | 10.98 |

As shown in Table 1, in all of Examples 1 to 4, it was confirmed that a polythiol composition (A) containing a polythiol component A1 as a main component could be obtained by decomposing a thiourethane resin with various decomposing agents.

### [Example 6]

### <Production of Formed body>

A formed body containing a thiourethane resin was obtained in the same manner as in Reference Production Example 1 (i.e., the polythiol composition (A) produced in Reference Production Example 3 was used as the polythiol composition (A)), except that the thickness between the pair of glass molds was changed from 9.0 mm to 2.5 mm.

Furthermore, a formed body containing a thiourethane resin was obtained in the same manner as above, except that the polythiol composition (A) produced in Reference Production Example 3 was changed to the polythiol compositions (A) produced in Examples 1 to 4.

### <Evaluation of Formed body>

Each of the molded bodies described above was evaluated as follows.

The results are shown in Table 2.

### (Yellow Index (YI), L*, a*, and b*)

The yellow index (YI), L*, a*, and b* of each formed body were measured using a SPECTROPHOTOMETER CM-5 manufactured by Konica Minolta, Inc.

### (Refractive Index (ne) and Abbe Number (ve))

Using a PULFRICH REFRACTOMETER KPR-30 manufactured by SHIMADZU CORPORATION, the refractive indices (ne, nF', nC') of the formed body were measured at 20°C at wavelengths of 546.1 nm (mercury e-line), 480.0 nm (Cd F' line), and 643.9 nm (Cd C' line). Based on these measurement results, the refractive index (ne) and the Abbe number (ve) of the formed body were determined.

### (Heat Resistance)

The glass transition temperature (Tg) of the formed body was measured by the TMA penetration method (load: 50 g; pin tip: 0.5 mmφ; heating rate: 10°C/min) using a THERMOMECHANICAL ANALYZER TMA-60 manufactured by SHIMADZU CORPORATION, and was used as an index of heat resistance.

### (Specific Gravity d)

The specific gravity d of the formed body was measured by the Archimedes method at 20°C.

**[Table 2]**

| | | Polythiol composition used as raw material for formed body (A) | | | | |
|---|---|---|---|---|---|---|
| | | Reference Production Example 3 | Example 1 | Example 2 | Example 3 | Example 4 |
| Evaluation results of formed body | Yellow Index | 0.65 | 0.72 | 0.88 | 1.45 | 0.75 |
| | L* | 95.46 | 95.46 | 95.44 | 95.21 | 95.44 |
| | a* | -0.10 | -0.13 | -0.14 | -0.21 | -0.11 |
| | b* | 0.51 | 0.55 | 0.60 | 0.88 | 0.54 |
| | Tg [°C] | 89.2 | 88.3 | 87.3 | 84.5 | 85.3 |
| | Refractive index ne | 1.665 | 1.665 | 1.665 | 1.663 | 1.665 |
| | Abbe number ve | 31 | 31 | 31 | 31 | 31 |
| | Specific gravity d | 1.36 | 1.36 | 1.36 | 1.35 | 1.36 |

As shown in Table 2, also in a case in which the polythiol compositions (A) obtained in Examples 1 to 4 were used as raw materials, it was confirmed that a formed body (i.e., resin formed body) having properties usable as an optical material (e.g., eyeglass lens) can be produced, as in a case in which the polythiol composition (A) obtained in Reference Production Example 3 was used as a raw material.

### [Example 6X]

In Example 6X, the same procedures as in Example 6 were carried out, except that the production of the formed body was changed as follows, and the same results as those in Example 6 (Table 2) were obtained.

### -Changes from Example 6-

In Example 6, m-xylylene diisocyanate (XDI) (52 parts by mass) was used in producing the formed body, but in Example 6X, the XDI (52 parts by mass) was changed to the XDI composition X1 (in an amount such that the amount of XDI contained was 52 parts by mass) as the XDI composition described above.

The XDI composition X1 was produced by adding trace amounts of compounds (N1), (N2), and (N3) to the main component XDI and mixing them.

For the XDI composition X1, gas chromatography measurements were performed under the above-described GC conditions 1 and 2. As a result,
the peak area of the compound (N1) corresponded to 0.20 ppm or more (specifically, 600 ppm) with respect to a peak area of 1 of the xylylene diisocyanate,
the peak area of the compound (N2) corresponded to 0.05 ppm or more (specifically, 18 ppm) with respect to a peak area of 1 of the xylylene diisocyanate, and
the peak area of the compound (N3) corresponded to 0.10 ppm or more (specifically, 100 ppm) with respect to a peak area of 1 of the xylylene diisocyanate.

### [Example 7]

### <Production of Polyamine Compound by Reaction of Polyurea Compound B1 Obtained in Example 1 with Ethylenediamine>

To a 25-mL PRESSURE-RESISTANT REACTION VESSEL manufactured by SAN-AI Kagaku Co. Ltd.,
the polyurea compound B1 (2.0 g; 9.0 mmol) obtained in Example 1 and ethylenediamine (3.2 g; 54 mmol) as a decomposing agent represented by Formula (3) were added, thereby obtaining a reaction solution.
Nitrogen gas was then filled into the reaction vessel, and the vessel was sealed.

Next, the reaction solution in the reaction vessel was heated to 145°C under a sealed condition, and stirred for 3 hours under a pressure condition at least 0.01 MPa higher than atmospheric pressure, thereby carrying out a reaction.

It was confirmed by gas chromatography (GC) that xylylenediamine (XDA) was generated as a polyamine compound by the reaction between the polyurea compound B1 and ethylenediamine.

The mass of the reaction solution after the reaction was 4.9 g, the concentration of XDA determined from the GC results was 18.6%, and the generation yield was 74.6% (the two-stage yield from thiourethane resin R1 was 68.2%).

After the reaction, the decomposing agent (ammonia) represented by Formula (1) generated by the decomposition of the polyurea compound B1 volatilized from the reaction solution and was easily removed. The decomposing agent represented by Formula (3) (ethylenediamine) can also be easily removed from the reaction solution after the reaction by distillation.

### [Example 8]

### <Production of Polyamine Compound by Reaction of Polyurea Compound B2 Obtained in Example 2 with Ethylenediamine>

The same procedures as in Example 7 were carried out, except that the polyurea compound B1 was changed to the polyurea compound B2 (2.0 g; 8.0 mmol) obtained in Example 2, and the amount of ethylenediamine was changed to 2.9 g (48 mmol).

It was confirmed by gas chromatography (GC) that xylylenediamine (XDA) was generated as a polyamine compound by the reaction between the polyurea compound B2 and ethylenediamine.

The mass of the reaction solution after the reaction was 4.6 g, the concentration of XDA determined from the GC results was 18.2%, and the generation yield was 75.6% (the two-stage yield from thiourethane resin R1 was 66.1%).

After the reaction, the decomposing agent (methylamine) represented by Formula (1) generated by the decomposition of the polyurea compound B2 volatilized from the reaction solution and was easily removed. The decomposing agent represented by Formula (3) (ethylenediamine) can also be easily removed from the reaction solution after the reaction by distillation.

### [Example 9]

### <Production of Polyamine Compound by Reaction of Polyurea Compound B3 Obtained in Example 3 with Ethylenediamine>

The same procedures as in Example 7 were carried out, except that the polyurea compound B1 was changed to the polyurea compound B3 (2.0 g; 7.2 mmol) obtained in Example 3, and the amount of ethylenediamine was changed to 2.6 g (43 mmol).

It was confirmed by gas chromatography (GC) that xylylenediamine (XDA) was generated as a polyamine compound by the reaction between the polyurea compound B3 and ethylenediamine.

The mass of the reaction solution after the reaction was 4.2 g, the concentration of XDA determined from the GC results was 19.3%, and the generation yield was 81.9% (the two-stage yield from thiourethane resin R1 was 77.8%).

The decomposing agent represented by Formula (3) (ethylenediamine) and the decomposing agent represented by Formula (1) (dimethylamine) generated by the decomposition of the polyurea compound B3 can be easily removed from the reaction solution after the reaction by distillation.

### [Example 10]

### <Production of Polyamine Compound Using Polycarbamate Compound C1 Obtained in Example 4>

The same procedures as in Example 7 were carried out, except that the polyurea compound B1 was changed to polycarbamate C1 (2.0 g; 79 mmol) obtained in Example 4, and the amount of ethylenediamine was changed to 2.9 g (48 mmol).

It was confirmed by gas chromatography (GC) that xylylenediamine (XDA) was generated as a polyamine compound by the reaction between polycarbamate C1 and ethylenediamine.

The mass of the reaction solution after the reaction was 4.9 g, the concentration of XDA determined from the GC results was 15.6%, and the generation yield was 70.4% (the two-stage yield from thiourethane resin R1 was 39.8%).

The decomposing agent represented by Formula (3) (ethylenediamine) and the decomposing agent represented by Formula (2) (methanol) generated by the decomposition of polycarbamate C1 can be easily removed from the reaction solution after the reaction by distillation.

### [Example 11]

### <Production of Polyamine Compound by Reaction of Polyurea Compound B1 Obtained in Example 1 with N,N'-Dimethylethylenediamine>

The same procedures as in Example 7 were carried out, except that 3.2 g (54 mmol) of ethylenediamine as the decomposing agent represented by Formula (3) was changed to 4.8 g (54 mmol) of N,N'-dimethylethylenediamine as the decomposing agent represented by Formula (3).

It was confirmed by gas chromatography (GC) that xylylenediamine (XDA) was generated as a polyamine compound by the reaction between the polyurea compound B1 and N,N'-Dimethylethylenediamine.

The mass of the reaction solution after the reaction was 6.7 g, the concentration of XDA determined from the GC results was 15.8%, and the generation yield was 85.5% (the two-stage yield from thiourethane resin R1 was 78.1%).

After the reaction, the decomposing agent (ammonia) represented by Formula (1) generated by the decomposition of the polyurea compound B1 volatilized from the reaction solution and was easily removed. The decomposing agent represented by Formula (3) (N,N'-dimethylethylenediamine) can also be easily removed from the reaction solution after the reaction by distillation.

### [Example 12]

### <Production of Polyamine Compound by Reaction of Polyurea Compound B1 Obtained in Example 1 with 2-Aminoethanol>

The same procedures as in Example 7 were carried out, except that 3.2 g (54 mmol) of ethylenediamine as the decomposing agent represented by Formula (3) was changed to 5.0 g (90 mmol) of 2-aminoethanol as the decomposing agent represented by Formula (3), the reaction temperature was changed from 145°C to 180°C, and the reaction time was changed from 3 hours to 6 hours.

It was confirmed by gas chromatography (GC) that xylylenediamine (XDA) was generated as a polyamine compound by the reaction between the polyurea compound B1 and 2-aminoethanol.

The mass of the reaction solution after the reaction was 6.7 g, the concentration of XDA determined from the GC results was 10.2%, and the generation yield was 55.7% (the two-stage yield from thiourethane resin R1 was 50.9%).

After the reaction, the decomposing agent (ammonia) represented by Formula (1) generated by the decomposition of the polyurea compound B1 volatilized from the reaction solution and was easily removed. The decomposing agent represented by Formula (3) (2-aminoethanol) can also be easily removed from the reaction solution after the reaction by distillation.

### [Example 13]

### <Production of Polyamine Compound by Reaction of Polyurea Compound B1 Obtained in Example 1 with Ethylene Glycol>

The same procedures as in Example 12 were carried out, except that 3.2 g (54 mmol) of ethylenediamine as the decomposing agent represented by Formula (3) was changed to 5.6 g (90 mmol) of ethylene glycol.

It was confirmed by gas chromatography (GC) that xylylenediamine (XDA) was generated as a polyamine compound by the reaction between the polyurea compound B1 and ethylene glycol.

The mass of the reaction solution after the reaction was 6.9 g, the concentration of XDA determined from the GC results was 7.1%, and the generation yield was 40.6% (the two-stage yield from thiourethane resin R1 was 37.1%).

After the reaction, the decomposing agent (ammonia) represented by Formula (1) generated by the decomposition of the polyurea compound B1 volatilized from the reaction solution and was easily removed. The decomposing agent represented by Formula (3) (ethylene glycol) can also be easily removed from the reaction solution after the reaction by distillation.

As described above, in Examples 7 to 13, a polyamine compound could be produced using a polyurea compound or a polycarbamate compound generated by the decomposition of a thiourethane resin as a raw material.

A polyisocyanate compound can be produced by reacting at least one of the produced polyamine compounds and the nitrates of these polyamine compounds with carbonyl dichloride.

A polymerizable composition can be produced by mixing the resulting polyisocyanate compound with an active hydrogen compound.

The obtained polymerizable composition can be used for producing a thiourethane resin or a urethane resin.

The disclosure of Japanese Patent Application No. 2022-135892, filed on August 29, 2022, is incorporated herein by reference in its entirety.

All publications, patent applications, and standards mentioned herein are herein incorporated by reference to the same extent as if each individual publication, patent application, or standard was specifically and individually indicated to be incorporated by reference.

## Claims

1. A method of producing a polythiol composition, the method comprising a generation step of generating a polythiol composition by decomposing a thiourethane resin using a decomposing agent represented by the following Formula (1) or (2): 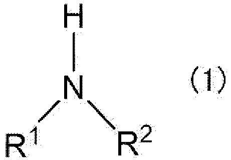
R³-OH (2)
wherein, in Formula (1), each of R¹ and R² independently represents a hydrogen atom, an alkyl group having from 1 to 3 carbon atoms, or an amino group, excluding a case in which R¹ and R² are both an amino group, and
in Formula (2), R³ represents an alkyl group having from 1 to 3 carbon atoms.

2. The method of producing a polythiol composition according to claim 1, wherein the generation step comprises reacting the thiourethane resin with the decomposing agent under a pressure higher than atmospheric pressure.

3. The method of producing a polythiol composition according to claim 1, which is a method of producing a polythiol composition for use in producing an optical material.

4. The method of producing a polythiol composition according to claim 1, wherein the thiourethane resin is recovered during at least one of a process of manufacturing eyeglass lenses, a process of manufacturing eyeglasses, or a process of disposing of eyeglasses.

5. A method of producing a polymerizable composition, the method comprising:
a step of producing a polythiol composition by the method of producing a polythiol composition according to any one of claims 1 to 4; and
a step of mixing the polythiol composition with a polyisocyanate compound, thereby obtaining a polymerizable composition containing the polythiol composition and the polyisocyanate compound.

6. The method of producing a polymerizable composition according to claim 5, wherein the step of obtaining a polymerizable composition is a step of mixing the polythiol composition with a polyisocyanate composition containing the polyisocyanate compound, thereby obtaining a polymerizable composition containing the polythiol composition and the polyisocyanate composition,
wherein the polyisocyanate composition contains:
a xylylene diisocyanate, and
at least one selected from the group consisting of the following compound (N1), the following compound (N2), and the following compound (N3), and
wherein, in a case in which the polyisocyanate composition contains the compound (N1), the compound (N1) has a peak area corresponding to 0.20 ppm or more with respect to a peak area of 100 of the xylylene diisocyanate in a measurement by high-performance liquid chromatography,
in a case in which the polyisocyanate composition contains the compound (N2), the compound (N2) has a peak area corresponding to 0.05 ppm or more with respect to a peak area of 100 of the xylylene diisocyanate in a measurement by high-performance liquid chromatography, or
in a case in which the polyisocyanate composition contains the compound (N3), the compound (N3) has a peak area corresponding to 0.10 ppm or more with respect to a peak area of 100 of the xylylene diisocyanate in a measurement by high-performance liquid chromatography:

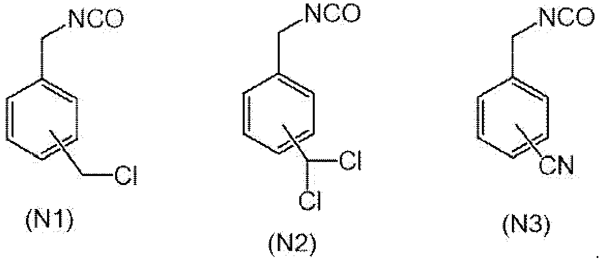

7. A method of producing a resin, the method comprising:
a step of producing a polymerizable composition by the method of producing a polymerizable composition according to claim 5; and
a step of obtaining a resin by curing the polymerizable composition.

8. A method of producing a polyamine compound, the method comprising:
a first step of generating a polyurea compound by decomposing a thiourethane resin using a decomposing agent represented by the following Formula (1); and
a second step of generating a polyamine compound by decomposing the polyurea compound using a decomposing agent represented by the following Formula (3): 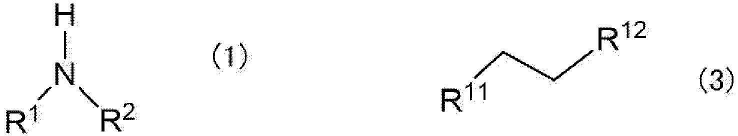
wherein, in Formula (1), each of R¹ and R² independently represents a hydrogen atom, an alkyl group having from 1 to 3 carbon atoms, or an amino group, excluding a case in which R¹ and R² are both an amino group, and
in Formula (3), each of R¹¹ and R¹² independently represents a hydroxy group, a mercapto group, an amino group, or a monomethylamino group.

9. The method of producing a polyamine compound according to claim 8, wherein the decomposing agent represented by Formula (3) is ethylenediamine, N,N'-dimethylethylenediamine, 2-aminoethanol, or ethylene glycol.

10. The method of producing a polyamine compound according to claim 8, wherein the first step comprises reacting the thiourethane resin with the decomposing agent represented by Formula (1) under a pressure higher than atmospheric pressure.

11. The method of producing a polyamine compound according to claim 8, wherein the second step comprises reacting the polyurea compound with the decomposing agent represented by Formula (3) under a pressure higher than atmospheric pressure.

12. A method of producing a polyamine compound, the method comprising:
a step X1 of generating a polycarbamate compound by decomposing a thiourethane resin using a decomposing agent represented by the following Formula (2) in the presence of a tertiary amine compound serving as a decomposition aid; and
a step X2 of generating a polyamine compound by decomposing the polycarbamate compound using a decomposing agent represented by the following Formula (3): 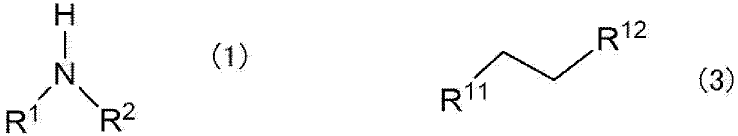
wherein, in Formula (2), R³ represents an alkyl group having from 1 to 3 carbon atoms, and
in Formula (3), each of R¹¹ and R¹² independently represents a hydroxy group, a mercapto group, an amino group, or a monomethylamino group.

13. The method of producing a polyamine compound according to claim 12,
wherein:
the tertiary amine compound has a molecular weight of 1000 or less, and
the decomposing agent represented by Formula (3) is ethylenediamine, N,N'-dimethylethylenediamine, 2-aminoethanol, or ethylene glycol.

14. The method of producing a polyamine compound according to claim 12, wherein the step X1 comprises reacting the thiourethane resin with the decomposing agent represented by Formula (2) under a pressure higher than atmospheric pressure.

15. The method of producing a polyamine compound according to claim 12, wherein the step X2 comprises reacting the polycarbamate compound with the decomposing agent represented by Formula (3) under a pressure higher than atmospheric pressure.

16. The method of producing a polyamine compound according to any one of claims 8 to 15, which is a method of producing a polyamine compound as a raw material for a polyisocyanate compound for use in producing an optical material.

17. The method of producing a polyamine compound according to any one of claims 8 to 15, wherein the thiourethane resin is recovered during at least one of a process of manufacturing eyeglass lenses, a process of manufacturing eyeglasses, or a process of disposing of eyeglasses.

18. A method of producing a polyisocyanate compound, the method comprising:
a step of producing a polyamine compound by the method of producing a polyamine compound according to any one of claims 8 to 15; and
a step of reacting at least one of the polyamine compound or a hydrochloride of the polyamine compound with carbonyl dichloride, thereby obtaining a polyisocyanate compound.

19. A method of producing polymerizable composition, the method comprising:
a step of producing a polyisocyanate compound by the method of producing a polyisocyanate compound according to claim 18; and
a step of mixing at least the polyisocyanate compound with an active hydrogen compound, thereby obtaining a polymerizable composition containing the polyisocyanate compound and the active hydrogen compound.

20. A method of producing a resin, the method comprising:
a step of producing a polymerizable composition by the method of producing a polymerizable composition according to claim 19; and
a step of obtaining a resin by curing the polymerizable composition.
